# EUROPEAN PATENT APPLICATION

(11) **EP 3 219 721 A1**
(43) Date of publication of application: **20.09.2017**
(21) Application number: 16305292.1
(22) Date of filing: 16.03.2016
(51) Int. Cl.: C07K 14/195, A61P 31/04, A61K 38/16

(54) **LISTERIOLYSIN S OR RELATED PEPTIDES AS ANTIBACTERIALS**

(71) Applicant: INSTITUT PASTEUR, 75015 Paris (FR)
(72) Inventor: PIZARRO-CERDA, Javier, 75013 PARIS (FR); QUEREDA-TORRES, Juan-José, 75013 PARIS (FR); COSSART, Pascale, 75015 PARIS (FR)
(74) Representative: Cabinet Plasseraud

(57) **Abstract**

The present invention relates to the use of Listeriolysin S (LLS) or related Streptolysin S (SLS)-like peptides as antibacterial peptides for therapeutic and non-therapeutic purposes.

SEQUENCE LISTING

<110>  INSTITUT PASTEUR

<120>  Listeriolysin S or related peptides as antibacterials

<130>  F226EP213

<160>  28

<170>  PatentIn version 3.5

<210>  1
<211>  23
<212>  PRT
<213>  Listeria monocytogenes

<400>  1

Cys Cys Ser Cys Ser Cys Ser Thr Cys Thr Cys Thr Cys Thr Cys Ala
1               5                   10                  15

Ser Ser Ala Ala Thr Lys Met
            20

<210>  2
<211>  30
<212>  PRT
<213>  Streptococcus pyogenes

<400>  2

Cys Cys Cys Cys Cys Thr Thr Cys Cys Phe Ser Ile Ala Thr Gly Ser
1               5                   10                  15

Gly Asn Ser Gln Gly Gly Ser Gly Ser Tyr Thr Pro Gly Lys
            20                  25                  30

<210>  3
<211>  24
<212>  PRT
<213>  Staphylococcus aureus

<400>  3

Cys Cys Ser Cys Ser Cys Ser Cys Ser Cys Ser Cys Ser Cys Ser Cys
1               5                   10                  15

Thr Ser Ala Ser Thr Ala Glu Gln
            20

<210>  4
<211>  33
<212>  PRT
<213>  Clostridium botulinum

<400>  4

Ser Cys Cys Cys Cys Ser Cys Cys Cys Cys Val Ser Val Ser Val Gly
1               5                   10                  15

Gly Gly Ser Ala Ser Thr Gly Gly Gly Ala Ala Ala Gly Gln Gly Gly
            20                  25                  30

Asn

<210>  5
<211>  49
<212>  PRT
<213>  Listeria monocytogenes

<400>  5

Met Asn Ile Lys Ser Gln Ser Ser Asn Gly Tyr Ser Asn Asn Ala Val
1               5                   10                  15

Gly Ser Glu Ala Met Asn Tyr Ala Ala Gly Cys Cys Ser Cys Ser Cys
            20                  25                  30

Ser Thr Cys Thr Cys Thr Cys Thr Cys Ala Ser Ser Ala Ala Thr Lys
        35                  40                  45

Met

<210>  6
<211>  53
<212>  PRT
<213>  Streptococcus pyogenes

<400>  6

Met Leu Lys Phe Thr Ser Asn Ile Leu Ala Thr Ser Val Ala Glu Thr
1               5                   10                  15

Thr Gln Val Ala Pro Gly Gly Cys Cys Cys Cys Cys Thr Thr Cys Cys
            20                  25                  30

Phe Ser Ile Ala Thr Gly Ser Gly Asn Ser Gln Gly Gly Ser Gly Ser
        35                  40                  45

Tyr Thr Pro Gly Lys
    50

<210>  7
<211>  50
<212>  PRT
<213>  Staphylococcus aureus

<400>  7

Met Met Lys Ile Asn Asn His Thr Ile Asn Gly Tyr Ser Asp Ile Asn
1               5                   10                  15

Ser Ser Glu Ala Met Gln Tyr Ala Ala Gly Cys Cys Ser Cys Ser Cys
            20                  25                  30

Ser Cys Ser Cys Ser Cys Ser Cys Ser Cys Thr Ser Ala Ser Thr Ala
        35                  40                  45

Glu Gln
    50

<210>  8
<211>  57
<212>  PRT
<213>  Clostridium botulinum

<400>  8

Met Leu Lys Phe Asn Glu His Val Leu Thr Thr Thr Asn Asn Ser Asn
1               5                   10                  15

Asn Lys Val Thr Val Ala Pro Gly Ser Cys Cys Cys Cys Ser Cys Cys
            20                  25                  30

Cys Cys Val Ser Val Ser Val Gly Gly Gly Ser Ala Ser Thr Gly Gly
        35                  40                  45

Gly Ala Ala Ala Gly Gln Gly Gly Asn
    50                  55

<210>  9
<211>  26
<212>  PRT
<213>  Listeria monocytogenes

<400>  9

Met Asn Ile Lys Ser Gln Ser Ser Asn Gly Tyr Ser Asn Asn Ala Val
1               5                   10                  15

Gly Ser Glu Ala Met Asn Tyr Ala Ala Gly
            20                  25

<210>  10
<211>  23
<212>  PRT
<213>  Streptococcus pyogenes

<400>  10

Met Leu Lys Phe Thr Ser Asn Ile Leu Ala Thr Ser Val Ala Glu Thr
1               5                   10                  15

Thr Gln Val Ala Pro Gly Gly
            20

<210>  11
<211>  26
<212>  PRT
<213>  Staphylococcus aureus

<400>  11

Met Met Lys Ile Asn Asn His Thr Ile Asn Gly Tyr Ser Asp Ile Asn
1               5                   10                  15

Ser Ser Glu Ala Met Gln Tyr Ala Ala Gly
            20                  25

<210>  12
<211>  24
<212>  PRT
<213>  Clostridium botulinum

<400>  12

Met Leu Lys Phe Asn Glu His Val Leu Thr Thr Thr Asn Asn Ser Asn
1               5                   10                  15

Asn Lys Val Thr Val Ala Pro Gly
            20

<210>  13
<211>  33
<212>  DNA
<213>  artificial sequence

<220>
<223>  synthetic oligonucleotide

<400>  13
ctgatcggat cccgctaaaa cgacggataa ttg                                33

<210>  14
<211>  33
<212>  DNA
<213>  artificial sequence

<220>
<223>  synthetic oligonucleotide

<400>  14
aaaaggcctc attcaaatgc ctccttttta ttt                                33

<210>  15
<211>  35
<212>  DNA
<213>  artificial sequence

<220>
<223>  synthetic oligonucleotide

<400>  15
aaaaggcctt aaattttaga atgaaaaagg gatac                                35


<210>  16
<211>  32
<212>  DNA
<213>  artificial sequence

<220>
<223>  synthetic oligonucleotide

<400>  16
gagtcagaat tcttgcccag ttttcaactt cc                                   32


<210>  17
<211>  20
<212>  DNA
<213>  artificial sequence

<220>
<223>  synthetic oligonucleotide

<400>  17
ggttcggttt ggattcaact                                                 20


<210>  18
<211>  20
<212>  DNA
<213>  artificial sequence

<220>
<223>  synthetic oligonucleotide

<400>  18
aactgctatc ccgtcacctg                                                 20


<210>  19
<211>  39
<212>  DNA
<213>  artificial sequence

<220>
<223>  synthetic oligonucleotide

<400>  19
aatgacccgg gtgtattgta atctagtgct tttgatttt                            39


<210>  20
<211>  50
<212>  DNA

<213>  artificial sequence

<220>
<223>  synthetic oligonucleotide

<400>  20
aaatatcatt cctttccaca aatatttttt gctgtttcct ttctatgtct          50


<210>  21
<211>  29
<212>  DNA
<213>  artificial sequence

<220>
<223>  synthetic oligonucleotide

<400>  21
aaatatttgt ggaaaggaat gatattttt          29


<210>  22
<211>  33
<212>  DNA
<213>  artificial sequence

<220>
<223>  synthetic oligonucleotide

<400>  22
tcgaggtcga cacactcata gcagccagtt tct          33


<210>  23
<211>  26
<212>  DNA
<213>  artificial sequence

<220>
<223>  synthetic oligonucleotide

<400>  23
atatactgtt tggctacttg gtatat          26


<210>  24
<211>  26
<212>  DNA
<213>  artificial sequence

<220>
<223>  synthetic oligonucleotide

<400>  24
aattcagctg tggaaacatt attttc          26


<210>  25
<211>  33
<212>  DNA
<213>  artificial sequence

<220>

<223>  synthetic oligonucleotide

<400>  25
ataacccggg ttttgatgct taagtggagt gaa                                    33


<210>  26
<211>  31
<212>  DNA
<213>  artificial sequence

<220>
<223>  synthetic oligonucleotide

<400>  26
ataagtcgac tgcacatgca cctcataact t                                      31


<210>  27
<211>  28
<212>  DNA
<213>  artificial sequence

<220>
<223>  synthetic oligonucleotide

<400>  27
ccatccaact cgagttttga tgcttaag                                          28


<210>  28
<211>  24
<212>  DNA
<213>  artificial sequence

<220>
<223>  synthetic oligonucleotide

<400>  28
cattcaaatg cctccttttt attt                                              24

## Description

The present invention relates to the use of Listeriolysin S (LLS) or related Streptolysin S (SLS)-like peptides as antibacterial peptides for therapeutic and non-therapeutic purposes.

Listeriolysin S (LLS) is a bacteriocin-like secreted hemolytic toxin and virulence factor reported to display cytotoxic activities, that is found only in a subset of *Listeria monocytogenes* strains from lineage I responsible for the majority of outbreaks of listeriosis (Cotter et al., PLoS Pathog., 2008, 4(9):e1000144).

Bioinformatic analysis suggests that LLS is post-translationally modified and belongs to the Streptolysin S (SLS)-like peptides, a family of Thiazole/Oxazole-modified peptides consisting of hemolytic and cytotoxic virulence factors produced by some Gram positive pathogenic bacteria including *Streptococcus pyogenes* (Group A *Streptococcus*) and related streptococci (Streptolysin S or SLS), *Staphylococcus aureus* (Staphylysin), *Clostridium botulinum* and *Clostridium sporogenes* (Clostridiolysin) and *Listeria monocytogenes* lineage I strains (Molloy et al., Nat. Rev. Microbiol., 2011, 9(9):670-681).

The LLS operon consists of a structural gene coding for a LLS precursor (*llsA*), three genes that are predicted to form a synthetase complex necessary for the maturation of the LLS (*llsB, llsY* and *llsD,* homologous to *sagBCD* in SLS operon), an ABC transporter (*llsG* and *llsH*), a putative protease (*llsP*) and a gene of unknown function (*llsX*).

By analogy with other SLS-like peptides, the LLS precursor (LlsA) is thought to consist of an N-terminal leader region, and a C-terminal core region with an extreme predominance of Cys, Ser and Thr residues as well as a putative Ala-Gly leader cleavage motif. The synthetase complex which comprises a dehydrogenase (LlsY), a cyclodehydratase (LlsB) and a docking protein (LlsD) is required for the post-translational conversion of Cys, Ser and/or Thr residues to thiazole, oxazole and/or methyloxazole heterocycles, respectively. Following modification, the amino-terminal leader sequence is believed to be cleaved from the mature core peptide by LlsP, resulting in a mature peptide product (LLS) that is exported across the bacteria membrane and secreted.

WO 2009/092790 relates to the use of Listeriolysin S as a cytotoxic agent for the treatment of cancer. WO 2009/092790 discloses that LLS or bacteria producing LLS are cytotoxic to a number of cell types including cancerous cells. WO 2009/092790 discloses also that LLS does not exhibit antimicrobial activity.

Here, the inventors have shown that, except for red blood cells, Listeriolysin S has no cytotoxic activity on mammalian cell lines from different origins. *In vivo,* the inventors have demonstrated instead that Listeriolysin S is a bacteriocin highly expressed in the intestine of orally infected mice, which alters the host intestinal microbiota and promotes intestinal colonization as well as deeper organ infection by *L. monocytogenes.* Further experiments from a bacterial collection of Gram positive and Gram negative bacteria, have demonstrated that Listeriolysin S has a narrow bactericidal activity. Listeriolysin S bacterial targets include in particular *L. monocytogenes* Listeriolysin S-negative strains, *Lactococcus lactis, Staphylococcus aureus* and some pathogenic strains of Streptococcus. Non-limiting examples of pathogenic strains of Streptococcus include: *S.pneumoniae, S.viridans* and *S.mutans,* causing dental pathologies; *S.agalactiae,* responsible for meningitis in newborn as well as disorders of pregnancy; *S.pyogenes,* causing tonsillitis or impetigo. This is the first report that a Streptolysin S-like peptide is a bacteriocin able to kill specifically other bacteria including pathogenic bacteria. Since Streptolysin S-like peptides are very similar in terms of structure and function, it can be expected that other Streptolysin S-likes are also bacteriocins which could enlarge the spectrum of pathogenic bacteria that can be targeted with Listeriolysin S.

Therefore, Listeriolysin S and related Streptolysin S-like peptides represent novel antibacterial agents with potential therapeutic and non-therapeutic applications. These new antibacterial peptides can be used in medicine as antibiotics to target specifically bacterial infections and diseases. They can also be used in food as additives to prevent food contamination or as disinfectants. Compared to other SLS-like peptides such as SLS or other bacteriocins such as Bacp222 (Wladyka et al., Scientific Reports, 2015, 5,14569-) which exhibit significant cytotoxic activities towards eukaryotic cells, Listeriolysin S has the advantage of having an antibacterial effect and a limited cytotoxic effect.

Therefore, the invention relates to the use of Listeriolysin S (LLS), a related Streptolysin S (SLS)-like peptide, or a combination thereof as antibacterial agent for therapeutic and non-therapeutic purposes.

According to the invention, Streptolysin (SLS)-like peptides include Listeriolysin S and related Streptolysin S-like peptides. In the context of the present invention, unless specified otherwise, "Streptolysin (SLS)-like peptide" or "peptide" refers indifferently to Listeriolysin S or a related Streptolysin S-like peptide.

In the following description, the standard one letter amino acid code is used.

The SLS-like peptide according to the invention comprises an amino acid sequence having at least 35 % identity, preferably at least 40, 45, 50, 55, 60, 65, 70, 75, 80, 85, 90, 95 or 98 % identity with SEQ ID NO: 1, said sequence comprising:
- a motif of at least 9 consecutive amino acids consisting of C, T, and S; C and S, or C and T residues, at its N-terminus, and
- at least one modified residue chosen from an oxazole- or 2-oxazoline-modified Serine (S) residue, a thiazole- or 2-thiazoline-modified Cysteine (C) residue and a methyloxazole- or 5-methyl-2-oxazoline-modified Threonine (T) residue.

### Definitions

In the following description:
- "a", "an", and "the" include plural referents, unless the context clearly indicates otherwise. As such, the term "a" (or "an"), "one or more" or "at least one" can be used interchangeably herein.
- The percent amino acid sequence identity is defined as the percent of amino acid residues in a Compared Sequence that are identical to the Reference Sequence after aligning the sequences and introducing gaps if necessary, to achieve the maximum sequence identity. Alignment for purposes of determining percent amino acid sequence identity can be achieved in various ways known to a person of skill in the art, for instance using publicly available computer software such as BLAST (Altschul et al., J. Mol. Biol., 1990, 215, 403-). When using such software, the default parameters, e.g., for gap penalty and extension penalty, are preferably used. For amino acid sequences, the BLASTP program uses as default a word length (W) of 3 and an expectation (E) of 10. The percent identity is determined on at least 20 consecutive amino acid residues of the reference sequence.
- "functional" refers to a biologically active peptide or protein or a polynucleotide encoding a biologically active peptide or protein. With regards to the peptide of the invention, functional refers to a peptide having antibacterial activity or a polynucleotide encoding a peptide having antibacterial activity.
- unless specified otherwise, "antibacterial" refers to bactericidal (to kill bacteria) or bacteriostatic (to stop the growth of bacteria).
- "individual" or "subject" refers to an individual susceptible to a bacterial disease.
- "bacterial disease" refers to a disease caused by infection with a pathogenic or opportunistic bacteria.
- unless specified otherwise, "treatment" refers to a preventive or prophylactic treatment that is administered prior to infection and/or a therapeutic treatment that is administered after infection with a pathogenic or opportunistic bacteria that causes a disease.

According to the invention, the 5-membered ring heterocycle carried by the modified S, C and T residues is formed with the side chain of said residues and the oxygen atom of the carbonyl group of the residue preceding directly said residues in the SLS-like peptide precursor or mature peptide.

The SLS-like peptides according to the invention encompass the well-known class of SLS-like peptides consisting of thiazole/oxazole-modified haemolytic exotoxins produced by pathogenic Gram positive bacterial species (review in Molloy et al., Nat. Rev. Microbiol., 2011, 9(9):670-681, for example) as well as functional variants thereof having antibacterial activity. SLS-like peptides include peptides encoded by the *llsA* gene of *Listeria monocytogenes* lineage I strains, by homologous genes from other pathogenic Gram positive bacteria or by functional mutant from these genes.

A gene locus comprising the SLS-like peptide gene and associated genes responsible for SLS-like peptide biosynthesis has been identified in some Gram-positive pathogenic bacteria (Lee et al., PNAS, 2008, 105 (15):5879-5884; Cotter et al., PLoS Pathog., 2008, 4(9):e1000144). The nucleotide sequences of these gene loci and the amino acid sequences of the corresponding proteins are well-known in the art and available in public data bases. Homologous genes from other pathogenic bacteria and corresponding proteins can be easily identified using standard sequence analysis software.

A *sagABCDEFGHI* gene cluster has been identified in *Streptococcus pyogenes, Streptococcus dysgalactiae subsp. equisimilis, Streptococcus iniae* and *Streptococcus equi.* SagA (Streptolysin S-associated gene A protein) is Streptolysin S precursor; SagBCD is the synthetase complex, comprising a cyclodehydratase (SagC), a dehydrogenase (SagB) and a docking protein (SacD); SagE is expected to be a membrane spanning peptidase, responsible for N-terminal leader sequence cleavage and required for viability; SagF is predicted to be membrane associated; SagG, SagH and SagI are thought to be membrane proteins that form an ABC type transporter.

The *sagABCDEFGHI* gene cluster corresponds to the locus tag spyM18_0799 to spyM18_0807 in *Streptococcus pyogenes* strain MGAS8232 genome sequence NCBI accession number NC_003485.1.

SagA corresponds to the amino acid sequence NCBI accession number WP_002985285.1 or SEQ ID NO: 6 (Table I). SagA (SLS precursor) comprises a N-terminal leader sequence (SEQ ID NO: 10) and a core peptide sequence (SEQ ID NO: 2) extremely rich in C and T residues which is post-translationally modified with Oxazole, Thiazole and/or Methyloxazole heterocycles derived from S, C and T residues, respectively, yielding mature SLS peptide. Site directed mutagenesis with alanine and proline suggests that oxazoles are formed at residues S34, S39, S46 and S48 and a thiazole is formed at C32 of the SagA propeptide (C9, S11, S16, S23 and S25 of SLS; Molloy et al., Nat. Rev. Microbiol., 2011, 9(9):670-681; Mitchell et al., J. Biol. Chem., 2009, 284:13004-13012). Liquid chromatography-tandem mass spectrometry (LC-MS:MS) with *in vitro* modified SLS has confirmed incorporation of oxazole moieties at S46 and S48 of SagA (Molloy et al., Nat. Rev. Microbiol., 2011, 9(9):670-681; Gonzalez et al., J. Biol. Chem.., 2010, 285 (36):28220-28228).

Another homologous gene cluster (*stsAGHBB'CC'DP*) has been identified in *Staphylococcus aureus.* StsA (Staphylysin S-associated gene A protein) is Staphylysin S precursor. StsBB'CC'D correspond to LlsBYD, StsP to LIsP and StsGH to LlsGH.

The *stsAGHBB'CC'DP* gene cluster corresponds to the locus tags SAB1381c to 1372 in *Staphylococcus aureus* strain RF122 genome sequence GenBank accession number AJ938182.1.

StsA corresponds to the amino acid sequence GenBank accession number CAI81070.1 or SEQ ID NO: 7 which comprises a leader sequence (SEQ ID NO: 11) and a core peptide (SEQ ID NO: 3) extremely rich in C, S and T residues which is post-translationally modified with Oxazole, Thiazole and/or Methyloxazole heterocycles derived from S, C and T residues, respectively, yielding mature Staphylysin peptide.

Another homologous gene cluster *closABCDEFGHI* has also been identified in *Clostridium botulinum* and *Clostridium sporogenes.* ClosA (Clostridiolysin S-associated gene A protein) is Clostridiolysin S precursor. ClosBCD correspond to SagBCD, ClosE to SagE, and ClosGHI to SagGHI.

The *closABCDEFGHI* gene cluster corresponds to the locus tag CLC_0560 to CLC_0568 in Clostridium botulinum A strain Hall genome sequence GenBank accession number CP000727.1.

ClosA corresponds to the amino acid sequence GenBank accession number ABS35961.1 or SEQ ID NO: 8 which comprises a leader sequence (SEQ ID NO: 12) and a core peptide (SEQ ID NO: 4) extremely rich in C and S residues which is post-translationally modified with Oxazole, Thiazole and/or Methyloxazole heterocycles derived from S, C and T residues, respectively, yielding mature Clostridiolysin peptide. Liquid chromatography-tandem mass spectrometry (LC-MS:MS) with *in vitro* modified Clostridiolysin has confirmed incorporation of methyloxazole moiety at T46 of ClosA (T22 of Clostridiolysin; Molloy et al., Nat. Rev. Microbiol., 2011, 9(9):670-681; Gonzalez et al., J. Biol. Chem.., 2010, 285 (36):28220-28228).

Functional variants or mutants are derived from wild-type amino acid or nucleotide sequences by the introduction of mutations (deletion(s), insertion(s) and/or substitutions(s)) at specific positions in said sequences. The mutations are advantageously mutations causing dispersed deletions and/or insertions of one to five amino acids in said amino acid sequences, amino acid substitutions, and/or N-or C-terminal deletion(s) of one or more amino acids in said sequences. The amino acid substitution(s) are advantageously chosen from conservative substitutions, i.e., substitutions of one amino acid with another which has similar chemical or physical properties (size, charge or polarity), which generally does not adversely affect the antibacterial properties of the protein/peptide. More preferably, said conservative substitution(s) are chosen within one of the following five groups: Group 1-small aliphatic, non-polar or slightly polar residues (A, S, T, P, G); Group 2-polar, negatively charged residues and their amides (D, N, E, Q); Group 3-polar, positively charged residues (H, R, K); Group 4-large aliphatic, nonpolar residues (M, L, I, V, C); and Group 5-large, aromatic residues (F, Y, W).

The invention encompasses peptides comprising or consisting of natural amino acids (20 gene-encoded amino acids in a L- and/or D-configuration) linked via a peptide bond as well as peptidomimetics of such protein where the amino acid(s) and/or peptide bond(s) have been replaced by functional analogues. Such functional analogues include all known amino acids other than said 20 gene-encoded amino acids. The invention also encompasses modified peptides derived from the above peptides by introduction of any further chemical modification into one or more amino acid residues, peptide bonds, N-and/or C-terminal ends of the peptide, as long as the modified peptide is functional. These modifications which are introduced into the peptide by the conventional methods known to those skilled in the art, include, in a non-limiting manner: the substitution of a natural amino acid with a non-proteinogenic amino acid (D amino acid or amino acid analog); the modification of the peptide bond, in particular with a bond of the retro or retro-inverso type or a bond different from the peptide bond; the cyclization, and the addition of a chemical group to the side chain or the end(s) of the peptide of the invention. These modifications may be used to increase the antibacterial activity, stability, and/or bioavailability of the peptide. For example, the peptide may be conjugated to a heterologous moiety, such as a tag or an agent of interest to increase its antibacterial activity, stability, and/or bioavailability or to facilitate its production.

The peptide according to the invention is a functional peptide having antibacterial activity. In particular, the peptide of the invention inhibits the growth of target bacteria *in vitro* and *in vivo.* The antibacterial activity of the peptide according to the invention can be readily verified by various assays, which are well-known to the person of ordinary skill in the art such as those described in the examples of the present Application. The antibacterial activity of the peptide can be assayed using either isolated peptide or host-cells, such as bacterial host-cells expressing the peptide as a fully processed and post-translationally modified biologically active mature peptide. *In vitro*, the number of viable bacteria (Colony-Forming-Unit or CFU) in a culture of target bacteria is reduced by at least 10 fold, preferably at least 100 fold, in the presence of the peptide of the invention compared to the same culture of target bacteria in the absence of said peptide. The antibacterial effect of the peptide according to the invention, *in vivo,* can be verified in appropriate animal models of bacterial diseases which are well-known in the art. The antibacterial effect of the peptide can be determined by measuring various parameters such as the reduction of objective clinical signs of the disease, the increase of survival, and/or the decrease of bacterial load in target organ(s).

In one embodiment, the SLS-like peptide according to the invention comprises:
at least 1, 2, 3, 4, 5, 6 or 7 thiazole- or 2-thiazoline-modified Cysteine residues, or a combination thereof;
at least 1, 2, 3 or 4 oxazole- or 2-oxazoline-modified Serine residues, or a combination thereof; and/or
at least 1, 2, 3 or 4 methyloxazole- or 5-methyl-2-oxazoline-modified Threonine residues, or a combination thereof.

In another embodiment, the SLS-like peptide according to the invention comprises:
at most 2, 3, 4, 5, 6, 7 or 8 thiazole- or 2-thiazoline-modified Cysteine residues, or a combination thereof;
at most 2, 3, 4 or 5 oxazole- or 2-oxazoline-modified Serine residues, or a combination thereof; and/or
at most 2, 3, 4 or 5 methyloxazole- or 5-methyl-2-oxazoline-modified Threonine residues, or a combination thereof.

In a preferred embodiment, the SLS-like peptide according to the invention comprises:
- 1 to 8, 2 to 7 or 3 to 6 of said modified Cysteine residues; for example 1 or 2; 2 or 3; 3 or 4; 4 or 5; 6 or 7; or 7 or 8 of said modified Cysteine residues, or a combination thereof;
- 1 to 5 or 2 to 4 of said modified Serine residues; for example 1 or 2; 2 or 3; 3 or 4; 4 or 5 of said modified Serine residues, or a combination thereof; and/or
- 1 to 5 or 2 to 4 of said modified Threonine residues; for example 1 or 2; 2 or 3; 3 or 4; 4 or 5 of said modified Threonine residues, or a combination thereof.

In another preferred embodiment, the SLS-like peptide according to the invention comprises:
- 6, 7 or 8, in particular 7 or 8 of said modified Cysteine residues, or a combination thereof;
- 3, 4 or 5, in particular 4 or 5 of said modified Serine residues, or a combination thereof; and/or
- 3, 4 or 5, in particular 4 or 5 of said modified Threonine residues, or a combination thereof.

In a more preferred embodiment of the SLS-like peptide according to the invention, all but one or all but two of the C, S and T residues of SEQ ID NO: 1 are thiazole- or 2-thiazoline-modified C residues, oxazole- or 2-oxazoline-modified S residues, and methyloxazole- or 5-methyl-2-oxazoline-modified Threonine (T) residues, respectively.

Alternatively, all of the C, S and T residues of SEQ ID NO: 1 are are thiazole- or 2-thiazoline-modified C residues, oxazole- or 2-oxazoline-modified S residues, and methyloxazole- or 5-methyl-2-oxazoline-modified Threonine (T) residues, respectively.

In another embodiment of the SLS-like peptide according to the invention, said at least one modified C, S or T residue is at position 1, 2, 3, 4, 5, 6, 7, 8, 9, 10, 11, 12, 13, 14, 15, 17, 18 or 21 of SEQ ID NO: 1. Preferably, said at least one modified C residue is at position 1, 2, 4, 6, 9, 11, 13 or 15 of SEQ ID NO: 1; said at least one modified S residue is at position 3, 5, 7, 17 or 18 of SEQ ID NO: 1; and/or said at least one modified T residue is at position 8, 10, 12, 14 or 21 of SEQ ID NO: 1.

In another embodiment of the SLS-like peptide according to the invention, said at least one modified S residue is an oxazole-modified S residue; said least one modified C residue is a thiazole-modified C residue; and/or said at least one modified T residue is a methyloxazole-modified T residue.

In another embodiment of the SLS-like peptide according to the invention, said motif starts at position 1 of SEQ ID NO: 1.

In another embodiment of the SLS-like peptide according to the invention, said motif comprises at least five C residues, preferably 6, 7, 8 or 9 Cysteine (C) residues.

In a preferred embodiment of the SLS-like peptide according to the invention, the one or two last C-terminal T or S residues of said sequence, preferably the last C-terminal T residue or the last two C-terminal S residues (last C-terminal S residue and penultimate one) are modified residues as defined above; preferably, the modified T residue is a methyloxazole-modified T residue and the modified S residue is an oxazole-modified S residue.

A preferred SLS-like peptide is a Listeriolysin S peptide (i.e. Listeriolysin (LLS) or a functional variant thereof). The LLS peptide comprises the amino acid sequence SEQ ID NO: 1 or an amino acid sequence having at least 70 % identity, preferably at least 75, 80, 85, 90, 95 or 98 % identity with SEQ ID NO: 1. The LLS peptide comprises advantageously a N-terminal motif of at least 9, preferably of 10, 11, 12, 13, 14 or 15 consecutive amino acids consisting of C, T, and S residues; preferably comprising at least 5 Cysteine residues, more preferably, 6, 7 or 8 Cysteine residues. The LLS peptide has at least one modified S, C or T residue as defined above for the SLS-like peptide according to the invention.

In a preferred embodiment of said LLS peptide, all but one or all but two of the C, S and T residues of SEQ ID NO: 1 are thiazole- or 2-thiazoline-modified C residues, oxazole- or 2-oxazoline-modified S residues, and methyloxazole- or 5-methyl-2-oxazoline-modified Threonine (T) residues, respectively; preferably thiazole-modified C residues, oxazole-modified S residues, and methyloxazole-modified Threonine (T) residues, respectively.

Alternatively, all of the C, S and T residues of SEQ ID NO: 1 are are thiazole- or 2-thiazoline-modified C residues, oxazole- or 2-oxazoline-modified S residues, and methyloxazole- or 5-methyl-2-oxazoline-modified Threonine (T) residues, respectively; preferably thiazole-modified C residues, oxazole-modified S residues, and methyloxazole-modified Threonine (T) residues, respectively.

Another preferred SLS-like peptide is a Streptolysine S (SLS) peptide (i.e. Streptolysin (SLS) or a functional variant thereof). The SLS peptide comprises the amino acid sequence SEQ ID NO: 2 or an amino acid sequence having at least 70 % identity, preferably at least 75, 80, 85, 90, 95 or 98 % identity with SEQ ID NO: 2. The SLS peptide comprises advantageously a N-terminal motif of 9 or more consecutive amino acids consisting of C and T residues; preferably comprising at least 5 C residues, more preferably, 6 or 7 C residues. The SLS peptide has at least one modified S, C or T residue as defined above for the SLS-like peptide according to the invention. Said at least one modified residue is advantageously a residue at position 9, 11, 16, 23 or 25 of said amino acid sequence SEQ ID NO: 2; preferably the residue at position 9 is a modified cysteine, and/or the residue at position 11, 16, 23 or 25 is a modified serine

In a first preferred embodiment of said SLS peptide, at least residues 23 and 25 are modified C, S or T residues, preferably modified S residues, more preferably oxazole-modified S residues. In a second preferred embodiment of said SLS peptide, at least residues 9, 11, 16, 23 and 25 are modified S, C or T residues, preferably the residue at position 9 is a modified cysteine, preferably a thiazole-modified C residue, and the residues at position 11, 16, 23 and 25 are modified serines, more preferably oxazole-modified S residues.

In a third preferred embodiment of said SLS peptide, the residues 9, 11, 16, 23 and 25 are modified S, C or T residues, preferably the residue at position 9 is a modified cysteine, preferably a thiazole-modified C residue, and the residues at position 11, 16, 23 and 25 are modified serines, more preferably oxazole-modified S residues.

In a fourth preferred embodiment of said SLS peptide, at most residues 9, 11, 16, 23 and 25 are modified S, C or T residues, preferably the residue at position 9 is a modified cysteine, preferably a thiazole-modified C residue, and the residues at position 11, 16, 23 and 25 are modified serines, more preferably oxazole-modified S residues.

Another preferred SLS-like peptide is a Staphylysin S peptide (i.e. Staphylysin S or a functional variant thereof). The Staphylysin S peptide comprises the amino acid sequence SEQ ID NO: 3 or an amino acid sequence having at least 70 % identity, preferably at least 75, 80, 85, 90, 95 or 98 % identity with SEQ ID NO: 3. The Staphylysin S peptide comprises advantageously a N-terminal motif of at least 9, preferably 10, 11, 12, 13, 14, 15, 16, 17 or 18 consecutive amino acids consisting of C, S or T residues; preferably comprising at least 5 C residues, more preferably, 6, 7, 8 or 9 C residues. The Staphylysin S peptide has at least one modified S, C or T residue as defined above for the SLS-like peptide according to the invention.

In the Staphylysin S peptide said at least one modified residue is advantageously a residue at position 1, 2, 3, 4, 5, 6, 7, 8, 9, 10, 11, 12, 13, 14, 15, 16, 17, 18, 20 or 21 of said amino acid sequence SEQ ID NO: 3. Preferably, the residue at position 1, 2, 4, 6, 8, 10, 12, 14 or 16 is a modified cysteine; the residue at position 3, 5, 7, 9, 11, 13, 15, 18 or 20 is a modified serine; and/or the residue at position 17 or 21 is a modified threonine residue as defined above.

In one embodiment, the Staphylysin S peptide according to the invention comprises:
at least 1, 2, 3, 4, 5, 6, 7 or 8 thiazole- or 2-thiazoline-modified C residues; in particular 1 to 9, 2 to 8, 3 to 7, 4 to 6 of said modified C residues; for example 1 or 2; 2 or 3; 3 or 4; 4 or 5; 6 or 7; 7 or 8, or 9 of said modified C residues, or a combination thereof;
at least 1, 2, 3, 4, 5, 6 or 7 oxazole- or 2-oxazoline-modified S residues; in particular 1 to 8, 2 to 7 or 3 to 6 ; for example 1 or 2; 2 or 3; 3 or 4; 4 or 5; 6 or 7; 7 or 8 of said modified S residues, or a combination thereof; and/or
at least 1 methyloxazole- or 5-methyl-2-oxazoline-modified Threonine (T) residues ; in particular 1 or 2 of said modified T residues, or a combination thereof.

In another embodiment, the Staphylysin S peptide according to the invention comprises:
at most 2, 3, 4, 5, 6, 7, 8 or 9 thiazole- or 2-thiazoline-modified C residues;
at most 2, 3, 4, 5, 6, 7 or 8 oxazole- or 2-oxazoline-modified S residues; and/or
at most 2 methyloxazole- or 5-methyl-2-oxazoline-modified Threonine (T) residues, or a combination thereof.

In another preferred embodiment, the Staphylysin peptide according to the invention comprises:
- 6, 7 or 8, in particular 7 or 8 of said modified C residues, or a combination thereof;
- 5, 6 or 7, in particular 6 or 7 of said modified S residues, or a combination thereof; and/or
- 1 or 2, in particular 2 of said modified T residues, or a combination thereof.

In a more preferred embodiment of the Staphylysin peptide according to the invention, all but one or all but two of the C, S and T residues of SEQ ID NO: 3 are thiazole- or 2-thiazoline-modified C residues, oxazole- or 2-oxazoline-modified S residues, and methyloxazole- or 5-methyl-2-oxazoline-modified Threonine (T) residues, respectively; preferably thiazole-modified C residues, oxazole-modified S residues, and methyloxazole-modified Threonine (T) residues, respectively.

Alternatively, all of the C, S and T residues of SEQ ID NO: 3 are are thiazole- or 2-thiazoline-modified C residues, oxazole- or 2-oxazoline-modified S residues, and methyloxazole- or 5-methyl-2-oxazoline-modified Threonine (T) residues, respectively; preferably thiazole-modified C residues, oxazole-modified S residues, and methyloxazole-modified Threonine (T) residues, respectively.

Another preferred Staphylysin S peptide according to the invention is a Staphylysin S peptide wherein said at least one modified residue comprises or consists advantageously of the last C-terminal T residue of its sequence (corresponding to T21 in SEQ ID NO: 3); the modified threonine residue is preferably a methyloxazole-modified threonine residue.

Yet another preferred SLS-like peptide is a Clostridiolysin S peptide (i.e. Clostridiolysin S or a functional variant thereof). The Staphylysin S peptide comprises the amino acid sequence SEQ ID NO: 4 or an amino acid sequence having at least 70 % identity, preferably at least 75, 80, 85, 90, 95 or 98 % identity with SEQ ID NO: 4. The Clostridiolysin S peptide comprises advantageously a N-terminal motif of at least 9, preferably 10, consecutive amino acids consisting of C and S; preferably comprising at least 5 C residues, more preferably, 6, 7 or 8 C residues. The Clostridiolysin S peptide has at least one modified S, C or T residue as defined above for the SLS-like peptide according to the invention. In a preferred embodiment of the Clostridiolysin S peptide according to the invention, said at least one modified residue comprises or consists advantageously of the last C-terminal T residue of its sequence (corresponding to T22 in SEQ ID NO: 4); the modified threonine residue is preferably a methyloxazole-modified threonine residue.

Advantageously, in the LLS, SLS, Staphylysin S or Clostridiolysin S peptide, said at least one modified S residue is an oxazole-modified S residue; said least one modified C residue is a thiazole-modified C residue; and/or said at least one modified T residue is a methyloxazole-modified T residue.

The peptide of the invention has an amino acid sequence comprising or consisting of SEQ ID NO: 1 or a functional variant thereof.

In some preferred embodiments, the peptide of the invention consists of up to 100 amino acids, preferably of up to 50 amino acids, more preferably of about 20 to about 35 amino acids.

In some other preferred embodiments, the peptide of the invention further comprises a leader sequence at its N-terminus. The leader sequence allows proper processing of the precursor peptide into a post-translationally modified biologically active mature peptide. Following post-translational modification of the core peptide by SagBCD synthetase complex (or an homolog or functional variant thereof), the leader sequence is cleaved by SagE (or an homolog or functional variant thereof), to release the mature peptide. The leader sequence is used for the biosynthesis or the *in vitro* synthesis of the peptide using SagBCD synthetase complex (or an homolog or functional variant thereof). The leader sequence is advantageously from the same bacteria species of as the peptide of the invention. In particular, the leader sequence is selected from the group consisting of the sequences SEQ ID NO: 9 to 12 corresponding to LlsA, StsA, SagA and ClosA leader sequence, respectively and the functional variants thereof having a sequence which is at least 70% identical, preferably at least 75, 80, 85, 90, 95 or 98 % identical to one of SEQ ID NO: 9 to 12.

In yet other preferred embodiments, the SLS-like peptide of the invention is fused at its N-or C-terminus to an heterologous peptide or protein moiety to form a fusion or chimeric protein. The length of the chimeric protein is not critical to the invention as long as the peptide is functional. The fusion protein is used to increase the stability, bioavailability, and/or allow the purification, detection, immobilization, production in expression systems, of the peptide of the invention. These moieties may be selected from: a binding moiety such as an epitope tag (polyHis6, FLAG, HA, myc.), a poly-lysine tag for immobilization onto a support, and a stabilization moiety (MBP or others). In addition, the peptide of the invention may be separated from the heterologous peptide/protein moiety by a linker which is long enough to avoid inhibiting interactions between the SLS-like peptide and the protein/peptide moiety. The linker may also comprise a recognition site for a protease, for example, for removing affinity tags and stabilization moieties from the purified chimeric protein according to the present invention.

In yet other preferred embodiments, the SLS-like peptide of the invention is complexed with a carrier molecule. The carrier molecule is a high-molecular mass molecule, such as non-ionic detergent, albumin, alpha-lipoprotein, lipoteichoic acid, RNase-resistant fraction of yeast RNA (RNA core) and the like. The peptide/carrier complex is useful in particular for the purification of the peptide of the invention.

The peptide of the invention is prepared by the conventional techniques known to those skilled in the art, in particular by solid-phase or liquid-phase synthesis, by expression of a recombinant DNA in a suitable cell system (eukaryotic or prokaryotic) or a combination of both techniques. More specifically, the peptide can be solid-phase synthesized, according to the Fmoc technique, originally described by Merrifield et al. (J. Am. Chem. Soc., 1964, 85: 2149-), and purified by reverse-phase high performance liquid chromatography; the peptide, can also be produced from the corresponding cDNAs, obtained by any means known to those skilled in the art; the cDNA is cloned into a eukaryotic or prokaryotic expression vector and the peptide produced in the cells modified with the recombinant vector is purified by any suitable means, in particular by affinity chromatography.

In particular, the peptide according to the invention is expressed from a host-cell carrying: (i) a *sagA* gene or a homolog or functional variant thereof and (ii) the associated genes necessary for the biosynthesis of the peptide as defined above, for example in an operon or in separate transcription units. The associated genes comprise at least the *sagBCD* genes of the synthetase complex, an homolog or functional variant thereof. In addition, the llsX and/or llsP genes may also be used for expressing LlsA and producing LLS. The host-cell is advantageously a prokaryotic cell, preferably a bacterial cell, such as for example *E. coli.* The *sagA* gene or homolog thereof is advantageously under the control of a constitutive promoter that is functional in the host cell. The *sagA* and associated genes may be the endogenous genes present in the bacteria or transgenes that have been introduced in the host cell by standard transformation methods and are transiently expressed in the host cell or incorporated in a stable extrachromosomal replicon, or integrated in the chromosome. The *sagA* and associated genes may be expressed as fusion proteins as described before. The SLS-like peptide is advantageously produced and/or purified from the host cells using the method disclosed in Alouf et al., Methods in Enzymology, 1988, 165, 59-64.

One aspect of the invention relates to a peptide according to the invention for use as an antibiotic. The peptide according to the invention is used in the treatment of a bacterial disease. In some embodiment, the disease is caused by a pathogenic or opportunistic bacteria. In some preferred embodiments, the pathogenic or opportunistic bacteria is a Gram positive bacteria, preferably chosen from Bacillales, Clostridia and Lactobacillales. The disease is advantageously caused by Staphylococcus spp., Streptococcus spp., Clostridium spp., Listeria spp., Bacillus spp., or Lactococcus spp. Staphylococcus spp. includes in particular *Staphylococcus saprophyticus, Staphylococcus aureus, Staphylococcus epidermidis*, and *Staphylococcus haemolyticus.* Streptococcus spp. includes in particular *Streptococcus pyogenes, Streptococcus pneumonia, Streptococcus viridans, Streptococcus agalactiae* and *Streptococcus mutants.* Clostridium spp. includes *Clostridium tetani* and *Clostridium perfringens.* Listeria spp. includes *Listeria innocua* and *Listeria monocytogenes.* Bacillus spp. includes *Bacillus cereus, Bacillus anthracis, Bacillus subtilis* and *Bacillus licheniformis.* Lactococcus spp. includes *Lactococcus lactis.*

A preferred use of the invention is the use of Listeriolysin S or a functional variant thereof for the treatment of an infection with a pathogenic or opportunistic bacteria, preferably chosen from the group comprising Staphylococcus spp., Streptococcus spp., Clostridium spp., Listeria spp and Lactococcus spp. In particular, Listeriolysin S or a functional variant thereof is advantageously used for treating infections with *Staphylococcus aureus,* Listeriolysin negative strains of *Listeria monocytogenes,* pathogenic strains *of Streptococcus* such as *Streptococcus pyogenes, Streptococcus pneumoniae, Streptococcus viridans, Streptacoccus agalactiae* and *Streptococcus mutants,* and *Lactococcus lactis.*

Another preferred use of the invention is the use of Clostridiolysin S or a functional variant thereof for the treatment of an infection with a pathogenic or opportunistic bacteria,

The peptide according to the invention may be used simultaneously, separately or sequentially with at least another pharmaceutical drug such as for example, an immunotherapeutic or anti-inflammatory agent, or another antimicrobial agent.

The peptide for use an antibacterial medicament is used in the form of a composition comprising a pharmaceutically acceptable vehicle, and eventually also another drug as defined above.

The pharmaceutical vehicles and carriers are those appropriate to the planned route of administration, which are well known in the art.

The composition of the invention comprises a therapeutically effective dose of peptide, sufficient to decrease the bacterial load in the target body compartment(s) or tissue(s) and thereby alleviate, relieve or cure the symptoms of the disease in the individual to whom it is administered.

The therapeutic effect of the composition according to the invention can be readily verified by various assays, using appropriate animal models of bacterial diseases which are known to the person of ordinary skill in the art. The effective dose is determined and adjusted depending on factors such as the composition used, the route of administration, the physical characteristics of the individual under consideration such as sex, age and weight, concurrent medication, and other factors, that those skilled in the medical arts will recognize.

The invention provides also a method for treating a bacterial infection or disease, comprising: administering to an individual a therapeutically effective amount of the composition as described above.

The composition of the present invention is generally administered according to known procedures used for antibiotherapy, at dosages and for periods of time effective to induce a therapeutic effect against the bacterial infection and associated bacterial disease in the individual. The administration may be by oral, sublingual, intranasal, rectal or vaginal administration, inhalation, or transdermal application. Preferably, the administration of the composition is oral.

In some embodiments, the pharmaceutical composition according to the invention is administered simultaneously, separately or sequentially, with at least another pharmaceutical drug as defined above.

In some embodiments, the peptide or composition according to the invention is used for the treatment of humans.

In other embodiments, the peptide or composition according to the invention is used for the treatment of animals such as pets (cats, dogs and others), livestock, poultry or others.

Another aspect of the invention relates to the use of a peptide according to the invention as antibacterial agent for non-therapeutic purposes. In particular, the peptide according to the invention can be used in food as an additive to prevent food contamination. Alternatively, the peptide according to the invention is used as a disinfectant.

The invention encompasses the use of several peptides of the invention, simultaneously, separately or sequentially.

Another aspect of the invention relates to an isolated Listeriolysin peptide as defined above.

The practice of the present invention will employ, unless otherwise indicated, conventional techniques which are within the skill of the art. Such techniques are explained fully in the literature.

**Table I: Peptide sequences**

| **Sequence name** | **SEQ ID NO:** | **Sequence** |
|---|---|---|
| Listeriolysin S (LLS) | 1 | CCSCSCSTCTCTCTCASSAATKM |
| Streptolysin S (SLS) | 2 | CCCCCTTCCFSIATGSGNSQGGSGSYTPGK |
| Staphylysin S | 3 | CCSCSCSCSCSCSCSCTSASTAEQ |
| Clostridiolysin S | 4 | SCCCCSCCCCVSVSVGGGSASTGGGAAAGQGGN |
| LlsA | 5 | MNIKSQSSNGYSNNAVGSEAMNYAAGCCSCSCSTCTCTCTCASSAATKM |
| SagA | 6 | MLKFTSNILATSVAETTQVAPGGCCCCCTTCCFSIATGSGNSQGGSGSYTPGK |
| StsA | 7 | MMKINNHTINGYSDINSSEAMQYAAGCCSCSCSCSCSCSCSCTSASTAEQ |
| ClosA | 8 | MLKFNEHVLTTTNNSNNKVTVAPGSCCCCSCCCCVSVSVGGGSASTGGGAAA GQGGN |
| LlsA leader sequence | 9 | MNIKSQSSNGYSNNAVGSEAM NYAAG |
| SagA leader sequence | 10 | MLKFTSNILATSVAETTQVAPGG |
| StsA leader sequence | 11 | MMKINNHTINGYSDINSSEAMQYAAG |
| ClosA leader sequence | 12 | MLKFNEHVLTTTNNSNNKVTAPG |

In addition to the above arrangements, the invention also comprises other arrangements, which will emerge from the description which follows, which refers to exemplary embodiments of the subject of the present invention, with reference to the attached drawings in which:

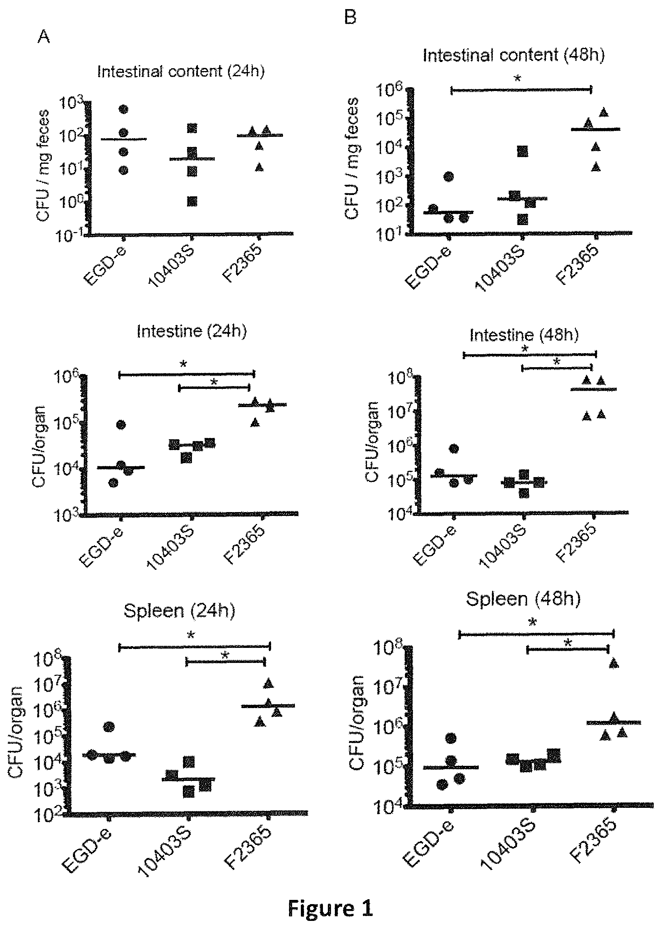

- **Figure 1****:** Comparative virulence of Lineage I strain F2365 and Lineage II strains EGD-e and 10403S. CFU counts in the intestinal content, intestine and spleen of Balb/c mice were measured at 24 h (A) and 48 h (B) after oral infection with 5 x 10⁹ CFU of the indicated strains. Each dot represents the value for one mouse. Statistically significant differences were evaluated by the Mann-Whitney test. *P < 0.05.

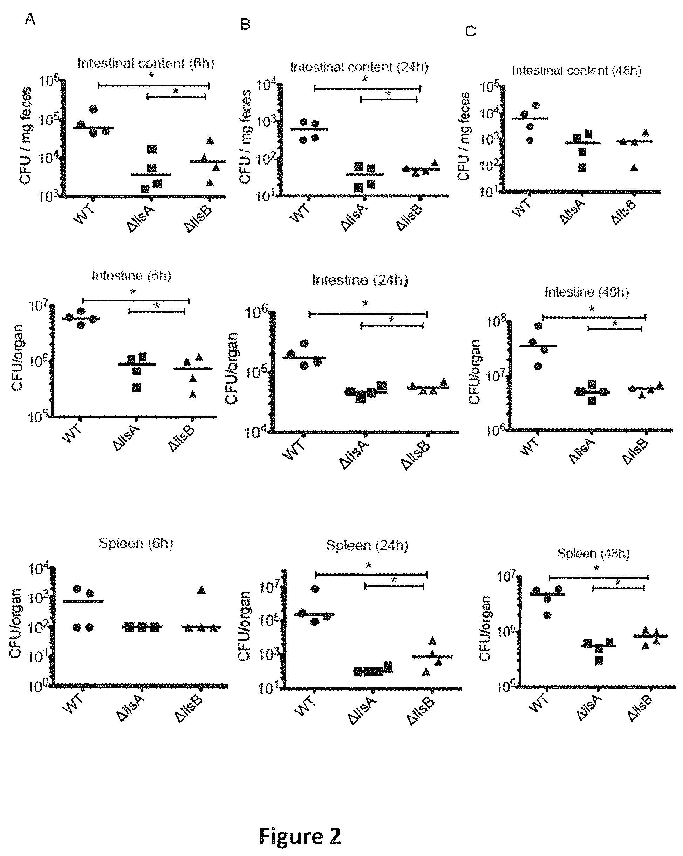

- **Figure 2****:** Importance of LLS for *in vivo* infection of a *L. monocytogenes* epidemic strain. Balb/c mice were injected orally with 5 x 10⁹ colony-forming units (CFU) of *L.monocytogenes* F2365 wt, Δ*llsA* or Δ*llsB.* Importantly, deletion of *llsB* renders LLS completely inactive in a blood agar test (Clayton et al., Appl. Environ. Microbiol., 2011, 77(1):163-171). CFU in the intestinal luminal content, the intestinal tissue (intestine) and spleen were assessed at 6 hours (A), 24 hours (B) and 48 hours (C) p.i.. Each dot represents the value for one mouse. Statistically significant differences were evaluated by the Mann-Whitney test. *P < 0.05. **P<0.01.

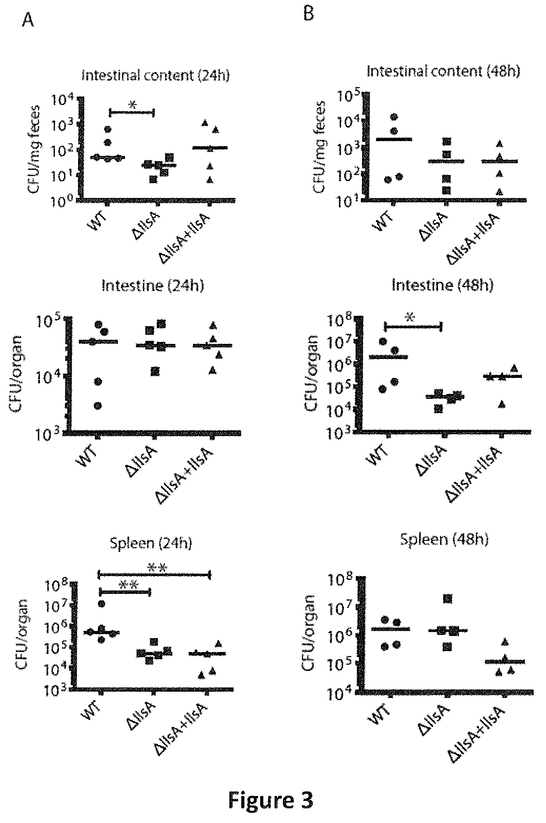

- **Figure 3**: Balb/c mice were injected orally with 5 x 10⁹ CFU of *L. monocytogenes* F2365 wt, Δ*llsA* and Δ*llsA*pPL2:*llsA.* CFU in the intestinal luminal content, the intestinal tissue (intestine) and spleen were assessed at 24 hours (A) and 48 hours (B) p.i. Four to five mice per group were used in each experiment. Each dot represents the value for one mouse. Statistically significant differences were evaluated by the Mann-Whitney test. *P < 0.05. **P < 0.01.

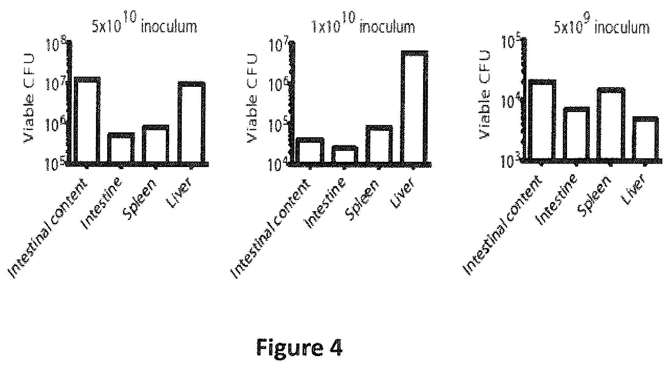

- **Figure 4**: Bacterial enumeration in the intestinal tissue (intestine), intestinal luminal content and liver at 96 h p.i. in Balb/c mouse infected with 5 x10¹⁰, 1 x 10¹⁰ and 5 x 10⁹ CFU *of L. monocytogenes* F2365*_{llsA:lux.}*

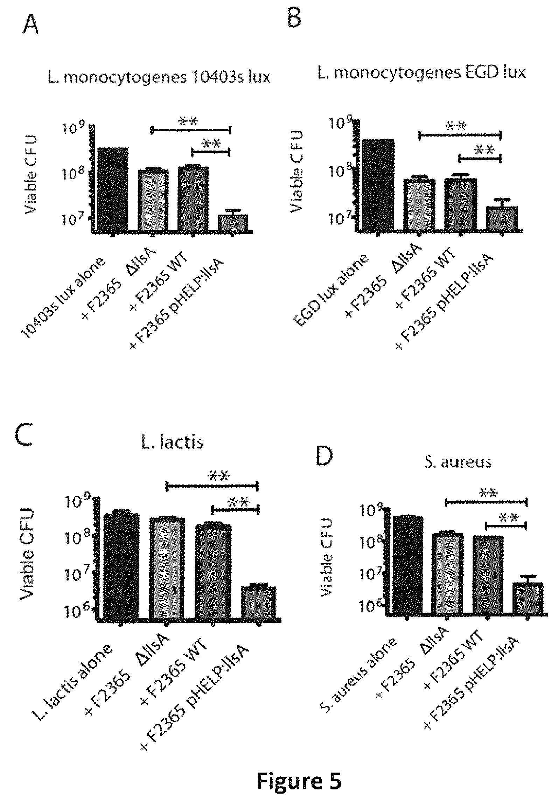

- **Figure 5****.** LLS inhibits the growth of other bacteria in vitro. Viable *L. monocytogenes* 10403Slux (A), *L. monocytogenes* EGDlux (B), *L. lactis* (C) and *S. aureus* (D) after 24 hours post-inoculation either alone or in co-culture with *L. monocytogenes* F2365 pHELP:LLS when compared to *L. monocytogenes* F2365 wt or *L. monocytogenes* F2365 Δ*llsA.* Data from three independent experiments are presented. Error bar shows standard deviation. Data was analyzed using a Student's t test. **, P<0.01.

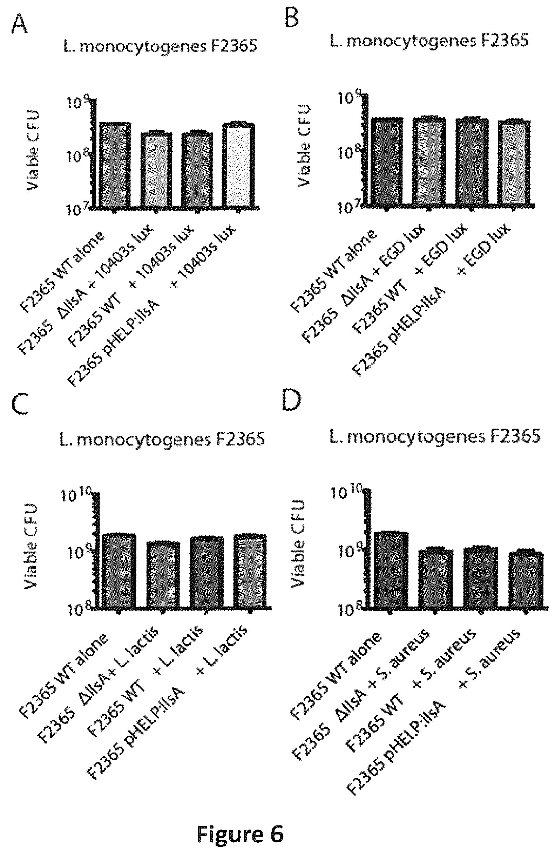

- **Figure 6****:** *L. monocytogenes* F2365 strains (wt, Δ*llsA* and pHELP:LLS) growth in single culture (alone) or in cocultures with *L. monocytogenes* 10403Slux (A), *L. monocytogenes* EGDlux (B), *L. lactis* (C) and *S. aureus* (D) after 24 hours post-inoculation. Data from three independent experiments are presented. Error bar shows standard deviation.

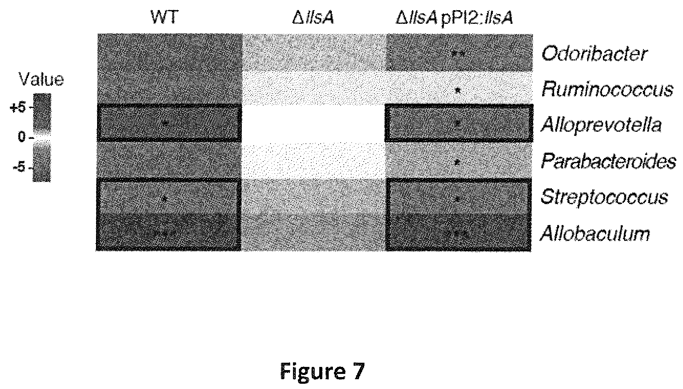

- **Figure 7****:** Intestinal microbiome analysis. Genera significantly altered in the gut microbiota of mice after 24h of infection with *L. monocytogenes* F2365 wt or *L. monocytogenes* F2365 Δ*llsA* pP12:*llsA* when compared to mice infected with *L. monocytogenes* F2365 Δ*llsA.* *P < 0.05, **P < 0.01, ***P < 0.001.

There will now be described by way of example a specific mode contemplated by the Inventors. In the following description numerous specific details are set forth in order to provide a thorough understanding. It will be apparent however, to one skilled in the art, that the present invention may be practiced without limitation to these specific details. In other instances, well known methods and structures have not been described so as not to unnecessarily obscure the description.

### Example 1: Materials and Methods

### Bacterial Strains and Growth Conditions

Bacterial strains and plasmids that we used in this study are listed in Table II.

**Table II: Strains and plasmids used in this study.**

| **Strain or Plasmid** | **Description** | **Reference** | **BUG / CIP number** |
|---|---|---|---|
| ***L. monocytogenes*** | | | |
| *L. monocytogenes* EGDe | Lineage II strain commonly used in laboratories (It lacks the LLS operon) | Becavin C, et al., MBio, 2014, 5(2):e00969-00914 | BUG1600 |
| *L. monocytogenes* F2365 | Lineage I strain associated with the 1985 listeriosis outbreak in California (It contains the LLS operon) | Linnan MJ, et al., N. Engl. J. Med., 1988, 319(13):823-828 | BUG 3012 |
| *L. monocytogenes* EGD | Lineage II strain commonly used in laboratories (It lacks the LLS operon) | Becavin C, et al., MBio, 2014, 5(2):e009b9-00914 | BUG 600 |
| *L. monocytogenes* 10403S | Lineage II strain commonly used in laboratories (It lacks the LLS operon) | Bishop et al., J. Immunol., 1987, 139(6):2005-2009 | BUG 1361 |
| *L. monocytogenes* F2365*^{llsA:lux}* | LLS promoter fused to a Lux reporter system in pPL2 lux | This study | BUG 3763 |
| *L. monocytogenes* F2365 Δ*llsA* | Deletion of the *llsA* gene | This study | BUG 3781 |
| *L. monocytogenes* F2365 Δ*llsA* pP12*:llsA* | *L. monocytogenes* F2365 Δ*llsA* expressing *llsA* under its natural promoter in the plasmid pPL2 | This study | BUG 3795 |
| *L. monocytogenes* F2365 pHELP:*llsA* | Synthetic strain that constitutively expresses LLS under the control of the pHELP promoter | This study | BUG 3817 |
| *L. monocytogenes* F2365 Δ*llsB* | Deletion of the *llsB* gene | This study | BUG 3672 |
| *L. monocytogenes* EGD^{lux} | *L. monocytogenes* EGD with the *hly* promoter fused to a Lux reporter system in pPL2 lux | Disson et al., Nature, 2008, 455(7216): 1114-1118 | |
| *L. monocytogenes* 10403S^{lux} | *L. monocytogenes* 10403S made luminescent | Hardy J, et al., Science 2004, 303(5659):851-853 | |
| | by chromosomal integration of a *lux kan* | | |
| | transposon cassette | | |
| ***E. coli* Top 10** | F- mcrA Δ(mrr-hsdRMS-mcrBC) ϕ80lacZΔM15ΔlacX74 recA1 araD139 Δ(ara-leu) 7697 galU galK rpsL (StrR) endAl nupG λ- | | Invitrogen, C4040 |
| *Staphylococcus aureus aureus* | Strains from the Institut Pasteur Collection | | CIP 65.8T |
| *Streptococcus thermophilus* | Strains from the Institut Pasteur Collection | | CIP 102303T |
| *Enterococcus faecalis* | Strains from the Institut Pasteur Collection | | CIP 103015T |
| *Acinetobacter johnsonii* | Strains from the Institut Pasteur Collection | | CIP 64.6T |
| *Lactococcus lactis lactis* | Strains from the Institut Pasteur Collection | | CIP 70.56T |
| *Lactobacillus paracasei paracasei* | Strains from the Institut Pasteur Collection | | CIP 103918T |
| *Lactobacillus paraplantarum* | Strains from the Institut Pasteur Collection | | CIP 104668T |
| *Lactobacillus plantarum plantarum* | Strains from the Institut Pasteur Collection | | CIP 103151T |
| *Lactobacillus rhamnosus* | Strains from the Institut Pasteur Collection | | CIP A157T |
| **Plasmids** pMAD | shuttle vector used for creating plasmids for mutagenesis | Arnaud et al., Appl. Environ. Microbiol., 2004, 70(11):6887-6891 | |
| pPL2 | Site-specific integration vector | Balestrino D, et al., Appl. Environ. Microbiol., 2010, 76(11):3625-3636 | |
| pPL2^{lux} | derivative of the vector pPL2 which harbors a synthetic *luxABCDE* operon | Bron et al., Appl. Environ. Microbiol., 2006, 72(4):2876-2884) | |
| pMAD-llsA | plasmid used to create the deletion Δ*llsA* mutant | This study | BUG 3751 |
| pMAD-llsB | plasmid used to create the deletion Δ*llsB* mutant | This study | BUG 3668 |
| pP12*:llsA* | *llsA* complementation plasmid | This study | BUG 3791 |
| pPL2^{*llsA*:lux} | pPL2*lux* in which the *llsA* promoter was fused to *luxABCDE* | This study | BUG 3755 |

| | | | |
|---|---|---|---|
| *BUG numbers are identification numbers of the "Unité des Interactions Bactéries Cellules" laboratory's *Listeria* strain collection. **CIP numbers are identification numbers of the "Centre Ressources Biologiques de L'Institut Pasteur". | | | |

*L. monocytogenes* F2365, *L. monocytogenes* EGD, *L. monocytogenes* 10403S, *S. aureus aureus, S. thermophilus, E.faecalis* and *A. johnsonii* were grown in Brain heart infusion broth (DIFCO). *L. lactis lactis, Lactobacillus paracasei paracasei, Lactobacillus paraplantarum, Lactobacillus plantarum plantarum* and *Lactobacillus rhamnosus* were grown in MRS broth (OXOID). *E. coli* was grown in LB broth. When required, antibiotics were added (chloramphenicol, 7 µg/ml for *Listeria* or 35 µg/ml for *E.col*i)*.* Unless otherwise indicated, bacteria were grown with shaking at 200 rpm in tubes at 37° C.

### Mutant construction

For the construction of the Δ*llsA and* Δ*llsB* deletion mutants, fragments of about 500-bp DNA flanking each gene were amplified by PCR using chromosomal DNA of *L. monocytogenes* F2365 as template and finally ligated into the thermo-sensitive pMAD using the BamHI/EcoRI or XmaI/SalI restriction sites (Arnaud et al., Appl. Environ. Microbiol., 2004, 70(11):6887-6891). PCR primers are listed in Table III.

**Table III: Oligonucleotides used in the present study**

| **Name** | **Sequence** | **Purpose** |
|---|---|---|
| **Oligonucleotides used to create mutants** | | |
| pMAD-llsA-A | ctgatcGGATCCcgctaaaacgacggataattg (SEQ ID NO:13) | Amplification of ∼500 bp fragment in 5' flanking region of *llsA* |
| pMAD-llsA-B2 | aaaAGGCCTcattcaaatgcctcctttttattt (SEQ ID NO:14) | Amplification of ∼500 bp fragment in 5' flanking region of *llsA* |
| pMAD-llsA-C2 | aaaAGGCCTtaaattttagaatgaaaaagggatac (SEQ ID NO: 15) | Amplification of ∼500 bp fragment in 3' flanking region of *llsA* |
| pMAD-llsA-D | gagtcaGAATTCttgcccagttttcaacttcc (SEQ ID NO: 16) | Amplification of ∼500 bp fragment in 3' flanking region of *llsA* |
| pMAD-llsA-X | ggttcggtttggattcaact (SEQ ID NO:17) | Verification of the Δ*llsA* deletion (mapping in reading frame) |
| pMAD-llsA-Y | aactgctatcccgtcacctg (SEQ ID NO:18) | Verification of the Δ*llsA* deletion (mapping in reading frame) |
| pMAD-llsB-A | aatgaCCCGGGtgtattgtaatctagtgcttttgatttt (SEQ ID NO:19) | Amplification of ∼500 bp fragment in 5' flanking region of *llsB* |
| pMAD-llsB-B | aaatatcattcctttccacaaatattttttgctgtttcctttcta tgtct (SEQ ID NO:20) | Amplification of ∼500 bp fragment in 5' flanking region of *llsB* |
| pMAD-llsB-C | aaatatttgtggaaaggaatgatattttt (SEQ ID NO:21) | Amplification of ∼500 bp fragment in 3' flanking region of *llsB llsB* |
| pMAD-llsB-D | tcgagGTCGACacactcatagcagccagtttct (SEQ ID NO:22) | Amplification of ∼500 bp fragment in 3' flanking region of *llsB llsB* |
| pMAD-llsB-X | atatactgtttggctacttggtatat ((SEQ ID NO:23) | Verification of the Δ*llsB* deletion |
| | | (mapping in reading frame) |
| pMAD-llsB-Y | aattcagctgtggaaacattattttc (SEQ ID NO:24) | Verification of the Δ*llsB* deletion (mapping in reading frame) |
| **Oligonucleotides used for Δ*llsA* complementation constructs** | | |
| pP12-llsAGHB-compF | ataaCCCGGGttttgatgcttaagtggagtgaa (SEQ ID NO:25) | Cloning of *llsA* for complementation assays |
| pP12-llsA-compR | ataaGTCGACtgcacatgcacctcataactt (SEQ ID NO:26) | Cloning of *llsA* for complementation assays |
| **Oligonucleotides used for *llsA* promoter insertion in a luciferase reporter system** | | |
| PllsAmonoFlong | ccatccaaCTCGAGttttgatgcttaag (SEQ ID NO:27) | Cloning of *llsA* promoter into pPL2^{lux} |
| 5'phospo | cattcaaatgcctcctttttattt (SEQ ID NO:28) | Cloning of *llsA* promoter into pPL2^{lux} |
| PllsAmonInbluntR | | |

| | | |
|---|---|---|
| Restriction sites in capital letters | | |

Allelic exchange was induced as previously described (Arnaud et al., Appl. Environ. Microbiol., 2004, 70(11):6887-6891). All PCR amplifications for cloning were performed using Phusion High fidelity polymerase and reagents (FINNZYMES, F-553) following the manufacturer's instructions. All plasmids and strains were confirmed by DNA sequencing. Quantitative real time PCR discarded any polar effect of the *llsA* and *llsB* deletions on the expression of genes located in their vicinity. To construct a pP12:*llsA* complementation plasmid, F2365 genomic DNA was used to amplify a fragment containing the *llsA* gene (oligos in table III) which was SalI-SmaI digested, and ligated into the pPL2 vector (Balestrino et al., Appl. Environ. Microbiol., 2010, 76(11):3625-3636).

### Mice infections

BALB/c mice were infected by oral gavage with 5x10⁹ CFU of the indicated strains. Mice were anesthetized before oral gavage with Isoflurane. The bacterial inoculum was prepared in a total volume of 200 µl. The bacterial inoculum was mixed with 300 µl CaCO3 50mg/ml before oral gavage. Bacterial numbers in the inoculums were verified by plating different dilutions onto Brain Heart Infusion (BHI) agar (BECTON DICKINSON) plates before and after injection. Mice were sacrificed at subsequent time points and intestines, spleens and livers were removed. The small intestine was opened and the luminal content was recovered in a 1.5 ml tube and weighted. The small intestine (duodenum, jejunum and ileum) tissue was washed three times in DMEM (Thermofisher), incubated for two hours in DMEM supplemented with 40 µg/ml gentamycin, and finally washed three times in DMEM. All the organs and the intestinal luminal content were homogenized, serially diluted and plated onto BHI plates or Oxford agar (OXOID) plates (intestinal content) and grown overnight at 37°C for 48 - 72 h. CFU were enumerated to assess bacterial load. Two independent experiments were carried out with 4-6 mice per group in each experiment. Statistically significant differences were evaluated by the Mann-Whitney test, and differences were considered statistically significant when p values were < 0.05. This study was carried out in strict accordance with the French national and European laws and conformed to the Council Directive on the approximation of laws, regulations and administrative provisions of the Member States regarding the protection of animals used for experimental and other scientific purposes (86/609/Eec). Experiments that relied on laboratory animals were performed in strict accordance with the Institut Pasteur's regulations for animal care and use protocol, which was approved by the Animal experiment Committee of the Institut Pasteur (approval number n°03-49).

### Evaluation of llsA promoter expression with a luciferase reporter system

The promoter of *llsA* was amplified using specific oligos PllsAmonoFlong and PllsAmonInbluntR (table III), digested with Xhol and cloned into SwaI-SalI digested pPL2*lux* as previously described (Bron et al., Appl. Environ. Microbiol., 2006, 72(4):2876-2884). The resultant plasmid pPL2*_{llsA:lux}* was isolated from *E. coli* and introduced into *L. monocytogenes* F2365 generating F2365*_{llsA:lux}*. For in vivo bioluminescence experiments, 8 week-old female Balb/c mice were orally inoculated with 5 x 10¹⁰, 1 x 10¹⁰ or 5 x 10⁹ *L. monocytogenes* F2365*_{llsA:lux}* grown in BHI broth to OD 1.0 at 37°C. Bioluminescence imaging was performed using a Xenogen IVIS 100 imager (XENOGEN) with a 5 min exposure time. Mice were anesthetized with Isoflurane. For CFU determinations, whole luminal content from intestine or cecum, intestinal tissue, cecum, liver and spleen were obtained, homogenized and serially diluted and plated on Oxford agar plates (OXOID). For in vitro bioluminescence experiments, overnight bacteria cultures were washed and resuspended in an equivalent volume of BHI containing the compounds to test, which included hydrogen peroxide (2 mM, 20 mM, 50 mM or 100 mM), trypsin, pepsin, mucin, NaHCO₃, or bile salts at different concentrations (from 10 mg/ml to 0.6 ng/ml using four fold dilutions). Triton™ X-100, TWEEN^{®} 20 and IPEGAL^{®} (SIGMA) were used in BHI at different concentrations ranging from 10% v/v to 0.01 % using 10 fold dilutions. Succinic acid, butiric acid, propionic acid, octanoid acid and ethanolamine (SIGMA) were assayed from 100 mM to 0.02 µM using four fold dilutions. Gastric fluid was prepared as previously described (Guinane et al., Appl. Environ. Microbiol. 2015, 81(22):7851-7859). Minimal media was prepared as described by Phan-Thanh and Gormon (Phan-Thanh L et al., Int. J. Food Microbiol., 1997, 35(1):91-95) but arabinose was added as only sugar source. Antibiotics used included: lincomycin, nalidixic acid, streptomycin, neomycin, doxycycline, vancomycin, rifampin, erythromycin, nisin, penicillin, trimethoprim, polymixin B, spectinomycin, ciprofloxacin and levofloxacin (SIGMA) at concentrations ranging from 100 µg/ml to 0.02 ng/ml using four fold dilutions. In vitro bioluminescence was investigated in a Xenogen IVIS 100 imager (XENOGEN) with a 5 min exposure time and with a plate-reading luminometer (Tristar LB 941, Berthold Technologies).

### Co-cultures assays

For co-culture experiments, 5 x 10⁷ CFU from overnight cultures were inoculated into 5 mL of fresh BHI either in single culture or in co-culture with another strain as indicated in figures and incubated statically at 37°C with 6% O₂. At 24 hours post-inoculation, cultures were serially diluted and plated on BHI and Oxford agar plates (OXOID). *L. monocytogenes* F2365 was differentiated from *L. monocytogenes* 10403_{S*lux*} and *L. monocytogenes* EGD*ₗᵤₓ* by bioluminescence imaging using a Xenogen IVIS 100 imager (XENOGEN). Experiments were carried out three times independently. Data was analyzed using Student's t test. Differences were considered statistically significant when p values were < 0.05.

### Constitutive expression of LLS

To generate a strain that constitutively expresses LLS, we put the *lls* genes under the control of the strong constitutive promoter pHELP (Riedel et al., Appl. Environ. Microbiol., 2007, 73(9):3091-3094). Briefly, pHELP was fused between two 500 nt DNA fragments flanking the start codon of *llsA.* This DNA construction was synthetically produced by gene synthesis (Eurofins Genomics) and cloned into *Sal*I *-* EcoRI restriction sites of thermo-sensitive pMAD vector. Mutagenesis was performed by double recombination as previously described (Arnaud et al., Appl. Environ. Microbiol., 2004, 70(11):6887-6891).

### Faecal microbiota analysis by 16S rRNA gene sequencing using Illumina technology

BALB/c mice were orally infected with 5 x 10⁹ CFU of each of the indicated strains (*L.monocytogenes* F2365 wt, Δ*llsA* and Δ*llsA*pPL2:*llsA*). Six mice were used for each strain. Eightweek old mice were kept together during 5 weeks prior to infection in order to homogenize their intestinal microbiota. Two days before infection each mouse was kept in an individual cage. Feces from each mouse were recovered two days and one day before infection as well at 6h and 24h p.i. Feces were kept at -20° C until DNA extraction was performed. 16S rRNA gene amplification was performed by using the Nextflex™ 16s v1 - v3 amplicon-seq kit. 16S fecal DNA was sequenced using Illumina® Miseq resulting in 287 000 ± 54 000 (mean ± sd) sequences of 300-base-long paired-end reads. Reads with a positive match with human or phiX phage were removed. Library adapters, primer sequences and base pairs occurring at 5' and 3' ends with a Phred quality score <20 were trimmed off using Alientrimmer (version 0.4.0) (Criscuolo et al., Genomics, 2013, 102(5-6):500-506). Filtered highquality reads were merged into amplicons with Flash (version 1.2.11) (Magoc et al., Bioinformatics, 2011, 27(21):2957-2963.). Resulting amplicons were clustered into OTUs with VSEARCH (version 1.4) (Rognes,https://github.com/torognes/vsearch). The process includes several steps for dereplication, singletons removal and chimera detection (when aligned against the ChimeraSlayer reference database). The clustering was performed at 97% sequence identity threshold producing 2090 OTUs. The OTU taxonomic annotation was performed with the SILVA SSU (version 123) database (Quast C, et al., Nucleic Acids Res., 2013, 41 (Database issue):D590-596.). The input amplicons were then mapped against the OTU set to get an OTU abundance table containing the number of reads associated with each OTU. The first two columns of this table (corresponding to the 2 pre-infection time points) were summed to strengthen the description of this initial state. The statistical analyses were performed with the R software version 3.1.1 and bioconductor packages version 2.14 (Gentleman et al., Genome Biol., 2004, 5(10):R80). The matrix of OTU count data was normalized at the OTU level using the normalization method included in the DESeq2 R package version 1.4.5 and described in (Anders et al., Genome Biol., 2010, 11(10):R106), as suggested in (MeMurdie et al., PLoS Comput. Biol., 2014, 10(4):e1003531). Normalized counts were then summed within genera to increase the power of the statistical analysis. The Generalized Linear Model implemented in the DESeq2 R package (Love et al., Genome Biol., 2014, 15(12):550) was then applied to detect differences in abundance of genera between time points for each listeria strain. A GLM model that included time, mice and listeria strains as main effects and interactions between time and strain as well as mice and strain was defined. The later interaction was useful to model the pairing between successive measurements coming from the same mice. Resulting p-values were adjusted according to the Benjamini and Hochberg (BH) procedure (Benjamini et al., Journal of the Royal Statistical Society. Series B (Methodological), 1995, 57(1): 289-300). The PCOA plot was performed with the ade4 R package version 1.7.2 and plots on figure 7 were generated with ggplot2 version 1.0.1.

### Cytotoxicity test

Human epithelial HeLa cells (ATCC CCL2), human trophoblastic Jeg3 cells (ATCC HTB36) and murine Raw264.7 macrophages (ATCC TIB71), grown and maintained in Dulbecco's modified Eagle's medium supplemented with 4.5 g/liter Glutamax (GIBCO) and 5% fetal bovine serum (GIBCO), were trypsinized (HeLa and Jeg3, GIBCO) or scraped (Raw264.7, TPP), plated onto glass coverslips (12 mm diameter, Fisher Scientific) in flat-bottom 24-well tissue culture plates (TPP) at a cell density of 1E5 cells per well and incubated for 18h at 37°C in a 10% CO₂ atmosphere.

Cells were then incubated either with partially purified Listeriolysin S (Cotter protocol: PLoS Pathogens 2008; 4 (9): e1000144) with purified Listeriolysin O (Glomski protocol: J Cell Biology 2002; 156(6): 1029-1038), with overnight grown washed *L.monocytogenes* EGD (ratio 100 bacteria:cell) or overnight grown washed *L.monocytogenes* F2365 pHELP:llsA*Δ*hly (ratio 100 bacteria:cell) for 15 min (purified LLS and LLO) or 1h (L.monocytogenes EGD and *L. monocytogenes* F2365 pHELP:llsAΔhly).

Cells were then washed in PBS (GIBCO), fixed for 20 min in 4% paraformaldehyde (Electron Microscopy Sciences), mounted on glass slides with Fluoromount G^{®} (Interchim) and observed with white light with a 63x apochromatic objective (Zeiss) under a Axiovert 200-M microscope (Zeiss). Images were taken using an EMCCD Neo Camera (Andor) piloted by Metamorph (Molecular Devices). Hemolytic activity of Listeriolysin S, Listeriolysin O, *L.monocytogenes* EDG and *L.monocytogenes* pHELP:llsAΔhly was confirmed using Columbia red blood agar plates (Biomerieux).

### Example 2: Listeriolysin S is a bacteriocin

### - LLS is a virulence factor for L. monocytogenes lineage I strains

To determine whether *L. monocytogenes* strains from lineage I are more virulent than *L.monocytogenes* strains belonging to lineage II, conventional Balb/c mice were orally infected with 5 x 10⁹ *L. monocytogenes* CFU of lineage I strain F2365 (responsible for the 1985 California outbreak (Linnan MJ, et al., N. Engl. J. Med., 1988, 319(13):823-828) and with the strains EGD-e and 10403S from lineage II and bacterial counts were evaluated in the intestinal content, the small intestine, spleen and liver at 24h and 48h post infection (p.i.). *L. monocytogenes* F2365 counts in the intestine and in the spleen at 24 h p.i. where significantly higher in mice infected with the F2365 strain than mice infected with the strains EGD-e and 10403S (Figure 1A). Interestingly, *L. monocytogenes* F2365 replicated and colonized the intestinal content at 48 h p.i.more efficiently than strains EGD-e and 10403S (Figure 1B). *L. monocytogenes* counts in the intestine and spleen at 48h p.i. were also significantly higher in the mice infected with the strain F2365 than mice infected with the EGD-e and 10403S strains (Figure 1B). These results showthat the *L. monocytogenes* lineage I strain F2365 is more virulent than lineage II strains in a mouse infection model.

### - LLS plays a critical role in L. monocytogenes survival within the gastrointestinal tract, thereby favoring organ colonization

To elucidate the role of LLS during the natural route of *Listeria* infection, mice were infected orally and first the persistence in the intestinal content of the epidemic F2365 wild type strain was compared to that of a Δ*llsA* and a Δ*llsB* isogenic mutant strain. LlsB is an enzyme which putatively performs post-translational modifications in the immature LLS peptide, and which deletion renders LLS completely inactive in a blood agar test (Cotter PD, et al., PLoS Pathog., 2008, 4(9):e1000144; Molloy et al., Nat. Rev. Microbiol. 2011, 9(9):670-681 ; Clayton et al., Appl. Environ. Microbiol., 2011, 77(1):163-171). In the murine oral infection model disclosed herein, the Δ*llsA* and Δ*llsB* mutant strains display significantly reduced bacterial loads in the intestinal content at 6 h p.i. compared to the wild-type strain, and these differences are also observed at 24 h and 48 h p.i., indicating that LLS plays a role in the persistence of *L. monocytogenes* within the intestinal lumen (Figure 2A, 2B and 2C). No significant changes in the feces or diarrhea between groups were observed. (. A complemented strain (Δ*llsA* pP12:*llsA*) was also tested and confirmed the results observed with the Δ*llsA* mutant, detecting lower counts of the Δ*llsA* strain in the intestinal content, in the small intestine and in the spleen when compared to the wild-type strain (Figure 3A and 3B). Complementation of Δ*llsA* mutant with a plasmid containing *llsA* under the control of its native promoter partially restored the virulence in the intestinal content and in the intestine (Figure 3A and 3B). However, the *llsA* complementation did not function in the spleen (Figure 3A and 3B). LLS expression is specifically triggered in the intestine which could explain the lack of *llsA* complementation in the spleen, where the metabolic cost of carrying a pP12 plasmid could be negative for bacterial fitness..

Together, these results demonstrate that LLS plays a critical role in *L. monocytogenes* survival within the gastrointestinal tract, thereby favoring organ colonization.

### - Except from red blood cells, Listeriolysin S has no cytolytic activity on mammalian cells from different origin

Cultures of human epithelial HeLa cells, human trophoblastic Jeg3 cells and murine Raw264.7 macrophages were incubated either with partially purified Listeriolysin S, with purified Listeriolysin O, or with overnight grown *L.monocytogenes* EGD (ratio 100 bacteria:cell), or overnight grown washed *L.monocytogenes* F2365 pHELP:llsA*Δ*hly (ratio 100 bacteria:cell) for 15 min (purified LLS and LLO) or 1h (L.monocytogenes EGD and *L*.*monocytogenes* F2365 pHELP:llsA*Δ*hly). Cells were then fixed and examined under the microscope to determine the presence or absence of cytotoxicity in the treated cells compared to the control.

**Table IV: Cytotoxicity Test**

| **Cells** | **Control** | ***L. monocytogenes* 3819 (LLS+++, no LLO)** | ***L.monocytogenes* 3651 (no LLS, LLO+++)** | ***L.monocytogenes* 3012 (no LLS, LLO+)** | **Purified LLO** | **Purified LLS** |
|---|---|---|---|---|---|---|
| **HeLa** | 0 | 0 | 0 | 0 | + | 0 |
| **Jeg3** | 0 | 0 | 0 | 0 | + | 0 |
| **Raw 264.7** | 0 | 0 | 0 | 0 | + | 0 |

The results show no morphological changes in any of the LLS treated cells compared to the control. By contrast a cytotoxic effect was observed in all LLO treated cells.

### - LLS is specifically, triggered in the intestine

Since LLS is not produced under routine laboratory growth conditions (Cotter PD, et al., PLoS Pathog., 2008, 4(9):e1000144) and its role during *in vivo* infection is unknown, the organs in which the LLS gene is activated in the mouse during oral infection were investigated. For this purpose, the LLS promoter was fused to a Lux reporter plasmid and integrated into the chromosome of the epidemic strain F2365 (F2365*llsA:lux*). Upon oral infection of conventional Balb/c mice with 5 x 10⁹ *L. monocytogenes* F2365*llsA:lux,* a strong bioluminescent signal is detected in the abdomen of infected animals as early as 7 hours postinfection. The bioluminescent signal is then continuously observed up to 96 h p.i. in the intestine specifically, and is stronger after abdominal skin and peritoneum dissection. Interestingly, no other organ (including liver and spleen, which are the main organs targeted by *L. monocytogenes*) displays bioluminescence on dissection despite the presence of bacteria (CFU determined by plating organ homogenates). To discard the possibility that the absence of bioluminescence in the liver and spleen could be due to a lower number of CFU in these organs compared to the intestine, Balb/c were orally infected with higher doses of F2365*llsA:lux,* namely 5 x 10¹⁰ and 1 x 10¹⁰ CFU per mouse. Mice orally infected with 5 x 10¹⁰ CFU yield approximately 1 x 10⁷ CFU in the liver at 96h p.i. and no bioluminescent signal was found in this organ in any of the mice analyzed (n=5). For comparison, mice displaying a bioluminescent signal in the intestine contained between 1 x 10⁴ and 1 x 10⁷ CFU in the intestinal luminal content and ≈ 1 x 10⁴ CFU in the intestinal tissue at 96 h p.i (Figure4). These results establish that the expression of LLS is specifically triggered in the intestine.

The LLS promoter induction could be attributed either to host derived signals, to intestinal microbiota signals, or a combination of both. To elucidate the potential contribution of the intestinal microbiota to the LLS activation, germ-free and conventional C57/BL6J mice were orally infected with 5 x 10⁹ *L. monocytogenes* F2365*llsA:lux.* Bioluminescent signals at 24h p.i. in the intestine of germ-free C57/BL6J mice were higher than in the conventional C57/BL6J mice. The lower bioluminiscent signal in the conventional C57/BL6J mice is due to the lower number of *L. monocytogenes* CFU in these mice compared with the germ-free ones, which confirms the role played by the microbiota in protecting the host during orally acquired listeriosis as previously described (Archambaud et al., Proc. Natl. Acad. Sci. USA, 2012, 109(41):16684-16689). These results show that the host produces signals that activate the LLS promoter. Next, different compounds or conditions found in the intestine that could be involved in transcriptional activation of the LLS promoter were examined using *L. monocytogenes F2365llsA:lux.* None of the compounds tested (mucin, gastric fluid (Guinane et al., Appl. Environ. Microbiol. 2015, 81(22):7851-7859), trypsin, pepsin, NaHCO3, bile salts, detergents, succinic acid, butyric acid, propionic acid, valeric acid, octanoid acid, ethanolamine and different antibiotics) or even 6% O₂ or intestinal content of mice added *ex vivo* could induce bioluminescence in *in vitro* conditions using the F2365*llsA:lux* strain. Furthermore, neither spent BHI with different concentrations of H₂O₂ or minimal medium with arabinose as only sugar source (high glucose levels inhibit expression of *Streptococcus anginosus* SLS (Asam et al., Med. Microbiol. Immunol., 2015, 204(2):227-237) induced expression of the lux operon.

### LLS is a bacteriocin

Next the contribution of LLS to the increased *L. monocytogenes* survival in the intestine of infected mice was investigated. The gastrointestinal tract is colonized by the largest microbial community of the body and previous observations suggested that LLS sequence and operon are related to the class I bacteriocins family (Cotter PD, et al., PLoS Pathog., 2008, 4(9):e1000144; Molloy et al., Nat. Rev. Microbiol. 2011, 9(9):670-681). It was thus hypothesized that LLS could behave as a bacteriocin.

First, the potential inhibitory activity of *L. monocytogenes* F2365 wt, *L. monocytogenes* F2365 Δ*llsA* and *L. monocytogenes* F2365 pHELP*:llsA* (a synthetic strain that constitutively expresses LLS) was examined against different bacterial species or bioluminiscent *L. monocytogenes* from other lineages that lack the LLS operon (Table II). Briefly, diluted overnight cultures of *L. monocytogenes* F2365 strains, and selected target strains were grown either alone or in co-culture for 24 hours in 6% 02 and viable CFUs were determined using either bioluminescence or plating on BHI or Oxford agar plates. After 24 hours of co-culture, there was a significant reduction in the growth of *L. monocytogenes* 10403Slux (a strain that lacks the LLS operon) (Figure 5A), *L. monocytogenes* EGDlux (a strain that lacks the LLS operon) (Figure 5B), *Lactococcus lactis* (Figure 5C) and *Staphylococcus aureus* (Figure 5D) when cocultured with *L. monocytogenes* F2365 pHELP*:llsA*. No growth differences were observed among the different *L. monocytogenes* F2365 strains when co-cultured with the LLS target strains (Figure 6). However, no effect of LLS was detected on *Streptococcus thermophilus, Lactobacillus* spp., *Enterococcus faecalis, Escherichia coli* or *Acinetobacter johnsonii.* These results show that LLS is the first reported *L. monocytogenes* bacteriocin able to inhibit the growth of other bacteria.

Since LLS is a bacteriocin, specifically produced in the intestine of orally infected mice and necessary for *L. monocytogenes* survival in this organ, it was reasoned that this toxin might influence the composition of the host intestinal microbiota and alter the intestinal environment, making this niche more beneficial to *L. monocytogenes.* To investigate this hypothesis, highthroughput 16S ribosomal DNA was used to examine the intestinal microbiota in mice orally infected with *L*. *monocytogenes* F2365 wt, *L. monocytogenes* F2365 Δ*llsA* and *L. monocytogenes* F2365 Δ*llsA* pP12*:llsA* at 6h and 24h p.i. Infection with *L. monocytogenes* F2365 resulted in alteration of the microbial community. An increase of *Firmicutes* phylum abundance was observed at 6h p.i. (wt P< 0.01, Δ*llsA* P<0.0001 and Δ*llsA* pP12*:llsA* P<0.05) which did not correlate with an increase in *L. monocytogenes.* Microbial community compositions of the intestine were different in mice orally infected with the *L. monocytogenes* F2365 wt and the *L. monocytogenes* F2365 Δ*llsA* pP12*:llsA* when compared to the *L. monocytogenes* F2365 Δ*llsA* strain at 24h p.i. (P<0.05 and P<0.01, respectively). A decrease in representatives of the *Allobaculum* (P<0.05 for the wt strain and P<0.0001 for the complemented strain Δ*llsA* pP12:*llsA*) and *Alloprevotella* (P<0.0001) genera was observed in mice infected with wt and Δ*llsA* pP12*:llsA* at 24h p.i. (Figure 7). *Allocabulum* genera include bacterial species that produce butyric acid and *Alloprevotella* genera include species that produce acetic acid (Greetham et al., Anaerobe, 2004, 10(5):301-307; Downes et al., Int. J. Syst. Evol. Microbiol., 2013, 63(4): 1214-1218). Butyrate is reported to inhibit virulence factor production in *L. monocytogenes* at the transcriptional level (Sun et al., J. Bacteriol., 2012, 194(19):5274-5284), while acetic acid was shown to inhibit *L*. *monocytogenes* growth (Ostling et al., J. Appl. Bacteriol., 1993, 75(1):18-24). Together, these data support a model in which LLS alters the host intestinal microbiota to increase *L. monocytogenes* persistence. Since the discovery of *L. monocytogenes* in 1926, its virulence factors have been shown to exert their activity by targeting host cells and tissues or by protecting the bacteria from host factors (Cossart P, Proc. Natl. Acad. Sci. USA, 2011, 108(49):19484-19491). The *L. monocytogenes* bile salt hydrolase (BSH) was the first factor described to be involved in survival within the intestinal lumen (Dussurget et al., Mol. Microbiol., 2002, 45(4):1095-1106). LLS was previously shown to be a hemolytic and cytotoxic factor associated (together with internalin A and genes related to teichoic acid biosynthesis) to infectious potential in a molecular epidemiological and comparative genomic study of *L. monocytogenes* strains from different lineages (Maury MM, et al., Nat. Genet., 2016; Cotter et al., PLoS Pathog., 2008, 4(9):e1000144).

In this study, the inventors demonstrate that LLS is a *L. monocytogenes* secreted bacteriocin, but also show that this virulence factor is specifically produced in the intestine of infected animals and targets distinct genera of the intestinal microbiota. As a consequence, *L. monocytogenes* lacking LLS is impaired in its capacity to compete with intestinal microbiota and does not survive as efficiently as wild type bacteria in the intestinal lumen, a critical step for the establishment of listeriosis. Interestingly, the reference strains EGDe and EGD which lack LLS can rarely cause human disease (Maury MM, et al., Nat. Genet., 2016). The mechanisms by which these strains can colonize the intestine need to be assessed in future experiments. A recent study showed that *Staphylococcus pseudintermedius* synthesizes BacSp222, a plasmid encoded peptide that behaves as a bacteriocin and as a cytotoxic factor against eukaryotic cells (Wladyka et al., Nature scientific reports, 2015, 5,14569-). Since *S. pseudintermedius* is a commensal in the skin and mucosal surfaces of domestic animals, BacSp222 secretion could help to outcompete commensal bacteria inhabitants in these niches (Wladyka et al., Nature scientific reports, 2015, 5,14569-). This example and the present work show how two different bacteria, *L. monocytogenes* and *S. pseudintermedius,* developed a common strategy to survive in the specific niches where they develop their pathogenic action: the intestine and the skin/mucosal surfaces, respectively. The results from the inventors show that the intestine is the main organ where LLS is expressed. This result does not discard that LLS could be expressed at lower levels not detectable by the bioluminiscent system used here in other organs as spleen and liver where a contribution of LLS in virulence after intraperitoneal infection was previously described (Cotter et al., PLoS Pathog., 2008, 4(9):e1000144).

The findings reported here could explain why LLS containing *L*. *monocytogenes* strains are those associated to the majority of epidemic outbreaks of listeriosis. The present results enhance our current understanding of epidemic listeriosis and highlight potential similarities to other enteric infectious diseases (Kommineni et al., Nature, 2015, 526(7575):719-722), paving the way for future studies to uncover the intestinal bacteria that control infection susceptibility of different groups within a population.

Furthermore, this is the first report that a Streptolysin S-like peptide is a bacteriocin able to kill specifically other bacteria including pathogenic or opportunistic bacteria. *In vitro* experiments show that, from a broad bacterial collection including Gram+ and Gram- bacteria, Listeriolysin S is active only against *Listeria monocytogenes* Listeriolysin S-negative strains, *Lactococcus lactis* and *Staphylococcus aureus*. *Staphylococcus aureus* is frequently found as part of the normal skin, nose and respiratory tract flora without inducing disease, but it is also one of the five common causes of hospital-acquired infections. *S. aureus* multidrug resistant strains are being increasingly isolated from hospital intensive-care units as well as from commercialized meat and poultry food products. *Lactococcus lactis* is considered as an opportunistic bacteria. Listeriolysin S is therefore a novel antibiotic with a very narrow bactericidal activity that could be used to target specifically infections with *S. aureus, Lactococcus lactis, Listeria monocytogenes* Listeriolysin S-negative strains or other pathogenic or opportunistic bacteria that can be potentially targeted specifically with Listeriolysin S. LLS could also be used as an additive or disinfectant to prevent food contamination by *L. monocytogenes, S. aureus* or other opportunistic or pathogenic bacteria that can be potentially targeted specifically with Listeriolysin. In addition, since Streptolysin S-like peptides are very similar in terms of structure and function, it can be expected that other Streptolysin S-like peptides are also bacteriocins which could enlarge the spectrum of opportunistic or pathogenic bacteria that can be targeted with LLS.

## Claims

1. Listeriolysin S or related peptide for use as an antibiotic, wherein said peptide comprises an amino acid sequence having at least 35 % identity with SEQ ID NO: 1, said sequence comprising :
- at its N-terminus, a motif of at least 9 consecutive amino acids consisting of Cysteine, Threonine, and Serine; Cysteine and Serine, or Cysteine and Threonine residues, and
- at least one modified residue chosen from an oxazole- or 2-oxazoline-modified Serine residue, a thiazole- or 2-thiazoline-modified Cysteine residue and a methyloxazole- or 5-methyl-2-oxazoline-modified Threonine residue.

2. The peptide for the use according to claim 1, which comprises:
- 1 to 8 thiazole- or 2-thiazoline-modified Cysteine residues, or a combination thereof;
- 1 to 5 oxazole- or 2-oxazoline-modified Serine residues, or a combination thereof; and/or
- 1 to 5 methyloxazole- or 5-methyl-2-oxazoline-modified Threonine residues, or a combination thereof.

3. The peptide for the use according to anyone of claim 1 or claim 2, wherein said at least one modified Cysteine, Serine or Threonine residue is at position 1, 2, 3,4, 5, 6, 7, 8, 9, 10, 11, 12, 13, 14, 15, 17, 18 or 21 of SEQ ID NO: 1.

4. The peptide for the use according to claim 3, wherein said at least one modified Cysteine residue is at position 1, 2, 4, 6, 9, 11, 13 or 15 of SEQ ID NO: 1; said at least one modified Serine residue is at position 3, 5, 7, 17 or 18 of SEQ ID NO: 1; and/or said at least one modified Threonine residue is at position 8, 10, 12, 14 or 21 of SEQ ID NO: 1.

5. The peptide for the use according to any one of claims 1 to 4, wherein all but one or all but two of the Cysteine, Serine and Threonine residues of SEQ ID NO: 1 are thiazole- or 2-thiazoline-modified Cysteine residues, oxazole- or 2-oxazoline-modified Serine residues, and methyloxazole- or 5-methyl-2-oxazoline-modified Threonine residues, respectively.

6. The peptide for the use according to any one of claims 1 to 4, wherein all of the Cysteine, Serine and Threonine residues of SEQ ID NO: 1 are thiazole- or 2-thiazoline-modified Cysteine residues, oxazole- or 2-oxazoline-modified Serine residues, and methyloxazole- or 5-methyl-2-oxazoline-modified Threonine residues, respectively.

7. The peptide for the use according to any one of claims 1 to 6, wherein said at least one modified Serine residue is an oxazole-modified Serine residue; said least one modified Cysteine residue is a thiazole-modified Cysteine residue; and/or said at least one modified Threoninr residue is a methyloxazole-modified Threonine residue.

8. The peptide for the use according to any one of claims 1 to 7, wherein said motif starts at position 1 of SEQ ID NO: 1.

9. The peptide for the use according to any one of claims 1 to 8, wherein said motif comprises at least five Cysteine residues.

10. The peptide for the use according to any one of claims 1 to 9, which comprises the amino acid sequence SEQ ID NO: 1 or an amino acid sequence having at least 70 % identity with SEQ ID NO: 1, said peptide comprising a N-terminal motif of 15 consecutive amino acids consisting of Cysteine, Threonine, and Serine residues and comprising eight Cysteine residues.

11. The peptide as defined in any one of claims 1 to 10, for use in the treatment of a bacterial disease caused by a pathogenic or opportunistic Gram positive bacteria.

12. The peptide for the use according to claim 11, wherein said peptide is a peptide as defined in claim 10 and said disease is caused by *Staphylococcus aureus,* Listeriolysin negative strains, *Listeria monocytogenes* or *Lactococcus lactis* or Streptococcus pathogenic strains.

13. Use of a peptide as defined in any one of claims 1 to 10, as antibacterial agent for non-therapeutic purposes.

14. The use of the peptide according to claim 13, as a food additive to prevent food contamination or as a disinfectant.

15. An isolated peptide as defined in claim 10.

16. The isolated peptide of claim 15, wherein all, all but one or all but two of the Cysteine, Serine and Threonine residues of SEQ ID NO: 1 are thiazole- or 2-thiazoline-modified Cysteine residues, oxazole- or 2-oxazoline-modified Serine residues, and methyloxazole- or 5-methyl-2-oxazoline-modified Threonine residues, respectively.
